(19) Europäisches Patentamt
European Patent Office
Office européen des brevets

(11) Publication number: 0 159 396 B1

(12) EUROPEAN PATENT SPECIFICATION

(45) Date of publication of patent specification: 27.06.90

(51) Int. Cl.$^5$: C 07 K 5/06, A 61 K 37/02

(21) Application number: 84112744.2

(22) Date of filing: 23.10.84

# (54) Carboxyalkyl peptide derivatives.

(30) Priority: 12.04.84 US 599307
18.10.84 US 662129

(43) Date of publication of application:
30.10.85 Bulletin 85/44

(45) Publication of the grant of the patent:
27.06.90 Bulletin 90/26

(84) Designated Contracting States:
BE CH DE FR GB IT LI NL SE

(56) References cited:
EP-A-0 126 974

(73) Proprietor: G.D. Searle & Co.
P.O. Box 1045
Skokie Illinois 60076 (US)

(72) Inventor: McCullagh, Keith
30 Manor Park Ave
Princess Risborough Buckinghamshire (GB)
Inventor: Wadsworth, Harry
82 St. Margarets Grove
Great Kingshill Buckinghamshire (GB)
Inventor: Hann, Michael
34 Brook Street
Watlington Oxon (GB)

(74) Representative: Werner, Hans-Karsten, Dr. et al
Deichmannhaus am Hauptbahnhof
D-5000 Köln 1 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 159 396 B1

**Description**

This invention relates to novel compounds having pharmacological activity, to the production thereof, to compositions containing them, and to their use in the treatment or management of conditions or diseases, e.g., rheumatoid arthritis, in which collagenase promoted collagen breakdown is a causative factor.

We have now found a group of compounds which act as inhibitors of mammalian collagenase which initiates collagen breakdown. Extensive proteolytic enzyme promoted degradation of articular cartilage is associated with joint distruction in rheumatoid arthritis. Collagen is one of the major components of the protein matrix of joint cartilage and bone. Histological observations of rheumatoid lesions have established that such lasions are characterized by the profilation of synovial lining cells with subsequent neovascularization and infiltration by lymphocytes, macrophages and plasma cells, collectively referred to as soft tissue or "pannus". The importance of such soft tissue in cartilage erosion has been well demonstrated.

Evason and coworkers, for example, found that large amounts of neutral collagenase are produced by pannus tissue (Evanson, J. M., et al,. Arthritis & Rheum., *27*, 2639—2651, 1968). More recently, others have confirmed that neutral collagenase plays an important degradative role in the arthritic joints of experimental animals (see Cambray, et al., Rheumatol Int. *1*, 11—16 and 17—20, 1981) and in humans (Cawston, et al., Arthritis & Rheum., *27*, 285—290, 1984).

A mono-specific antiserum to purified synovial collagenase has been used to localize the enzyme in rheumatoid tissues [Wooley, et al., Eur. J. Biochem., *69*, 421—428, 1976]. Immunoreactive collagenase was detected in high density at the cartilage-pannus junction — the site of joint erosion — but not in the cartilage matrix or synovial tissue remote from the cartilage-pannus junction [Wooley, et al., Arthritis & Rheumatism, *20*, 1231—1349.] Wooley, et al., (Science, *200*, 773—775, 1978) have further identified a subpopulation of synovial cells responsible for collagenase production.

Thus, the foregoing observations have provided conclusive evidence that collagenase is directly involved in the cartilage erosion process seen in rheumatoid arthritis. Accordingly, the compounds of the present invention which specifically inhibit mammalian collagenase are pharmacologically useful in the treatment of rheumatoid arthritis and related diseases in which collagenolytic activity is a contributing factor, such as corneal ulceration, peridontal disease, tumor invasion, dystrophic epidermolysis bullosa, etc.

These compounds have substantially no angiotensin converting enzyme (ACE)-inhibiting activity. ACE inhibitors are described in EP—A—0012401 ACE is a carboxydipeptidase — it cleaves a peptide substrate two residues from the C-terminus. Consequently the C-terminal carboxylic acid is a prime recognition site for both substrates and inhibitors; removal of this ionic binding group drastically reduces inhibitory potency. Collagenase, on the other hand, is an endopeptidase and, as such, has not prerequisite for this binding interaction. Additionally, the structure of collagen differs essentially from angiotensin-I which is a decapeptide and is cleaved at a phenylalanine-histidine bond to give an octapeptide (angiotensin-II) and a dipeptide (histidylleucine). Collagen is much more complex, in being a triple helix, each strand of the helix containing of the order of 1,000 amino acid residues, the sequence of amino acids around the site cleaved by collagenase being completely different from that around the cleavage site of Angiotensin I. Collagenase cleaves this triple helix at a single locus on each chain approximately two-thirds of the way along the chain from the N-terminus. The amide bond which is cleaved by collagenase is either a glycine-leucine or a glycine-isoleucine bond.

The present invention provides compounds of the general formula I:

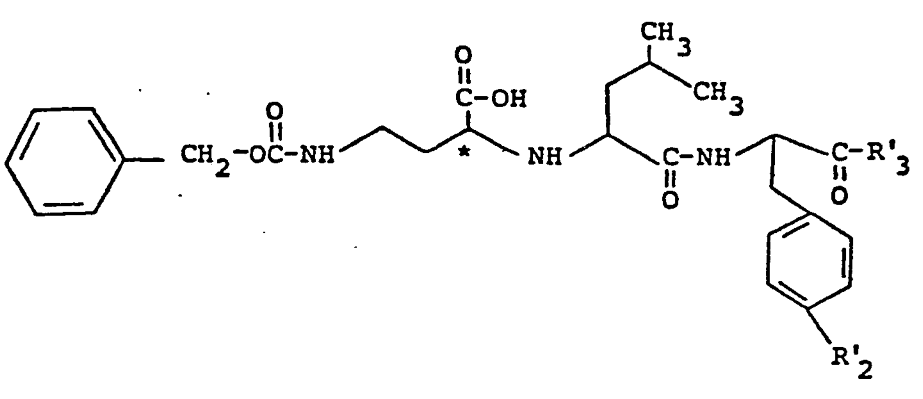

and the pharmaceutically acceptable acid addition salts thereof wherein $R'_2$ represents hydroxy, straight or branched chain alkoxy of from 1 to 10 carbon atoms, cycloalkoxy of from 3 to 10 carbon atoms, aralkoxy

comprising phenyl or naphthyl and an alkyl portion of from 1 to 4 carbon atoms or substituted alkoxy wherein the substituent is selected from alkylaminocarbonyl or the group

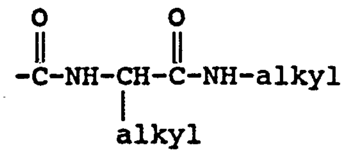

and $R'_3$ represents methylamino or phenylethylamino; and the stereochemistry of the carbon atom marked by the asterisk is R or S or a diastereometric mixture thereof, with R configuration being preferred, excluding N-(3-N-(benzyloxycarbonyl)amino-1-(R)-carboxypropyl)-L-leucyl-O-methyl-L-tyrosine N-methylamide.

The latter compound was proposed in EP—A—0 126 974 which forms part of the state of the art pursuant to Article 54 (3) EPC.

The term alkyl as used herein to designate a group or a part thereof includes reference to both straight and branched alkyl groups and to cycloalkyl groups which may contain from 1 to 10, preferably 1 to 6, carbon atoms in the case of straight or branched chain non-cyclic alkyl groups (for example methyl, ethyl, propyl, isopropyl) and from 3 to 10, preferably 3 to 7 in the case of cyclic alkyl groups (for example cyclopentyl, norbornyl).

By the term aryl, is meant phenyl or naphthyl.

The terms aralkyl and aralkoxy include in particular those groups containing 1 to 4 carbon atoms in the alkyl portion, and those groups in which aryl has the meaning just given.

Typical pharmaceutically acceptable addition salts are those derived from mineral and organic acids such as hydrochloric, hydrobromic, hydroiodic, p-toluene sulphonic, sulphuric, perchloric, acetic, benzoic and trifluoroacetic.

There are several chiral centres in the compounds according to the invention because of the presence of asymmetric carbon atoms. These centres may be racemised or in any optically active form, as well as comprise R, S or diastereomeric mixture.

The compounds according to the invention exhibit inhibitory action against collagenase. This was determined following the procedure of Cawston and Barret, *Anal. Biochem.*, *99*, 340—345 (1979) whereby the 1 mM of the inhibitor being tested or dilutions thereof are incubated at 37°C for 16 hours with native collagen and collagenase (buffered with Tris HCl—$CaCl_2$; pH 7.6). The collagen is acetyl $^{14}C$ collagen. The samples are centrifuged to sediment undigested collagen and an aliquot of the radiactive supernatant removed for assay on a scintillation counter as a measure of hydrolysis. The collagenase activity in the presence of 1 mM inhibitor, or a dilution thereof, is compared to activity in a control devoid of inhibitor and the results reported as that inhibitor concentration effecting 50% inhibition of the collagenase. Table II illustrates the activity of compounds of this invention.

For use in treatment of rheumatoid arthritis the compounds of this invention can be administered by any convenient route preferably in the form of a pharmaceutical composition adapted to such route and in a dose effective for the intended treatment. In the treatment of arthritis administration may conveniently be by the oral route or by injection intraarticularly into the affected joint. The daily dosage for a 70 kilogram mammal will be in the range of 10 milligrams to 1 gram.

The compounds of this invention can be formulated in compositions such as tablets, capsules or elixirs for oral administration or in sterile solutions or suspensions for parenteral administration. About 10 to 500 mg of a compound according to the invention is compounded with a physiologically acceptable vehicle, carrier, excipient, binder, preservative, stabilizer, flavour, in a unit dosage from as called for by accepted pharmaceutical practice. (See for example, Remington's Pharmaceutical Science Mach Publishing Co., Easton, Penn, 1965). The amount of active substance in these compositions or preparations is such that a suitable dosage in the range indicated is obtained.

The compounds according to the invention may be made by methods which are generally known in peptide chemistry of analogous compounds. In particular it is to be understood that reactive groups not involved in a particular reaction (e.g. amino, carboxy, hydroxy) may be protected by methods standard in peptide chemistry prior to reactions of other groups and subsequently deprotected.

The intermediates of use in the production of the end-products are either known compounds or can be made by known methods, as described in the Examples.

In order that the invention may be more fully understood the following examples are given. All temperatures herein are degrees C.

Example 1 (Reference)

N-[3-N-(Benzyloxycarbonyl)amino-1-(R)-carboxypropyl]-L-leucyl-O-methyl-L-tyrosine N-Methylamide

This was prepared in two stages from methyl 4-N-(benzyloxycarbonyl)amino-2-bromo-butanoate and L-leucyl-O-methyl-L-tyrosine N-methylamide as described below:

(a) N-[3-N-(Benzyloxycarbonyl)amino-1-(R)-methoxy-carbonylpropyl]-L-leucyl-O-methyl-L-tyrosine N-Methylamide

Methyl 4-N-(benzyloxycarbonyl)amino-2-bromo-butanoate (30 g), L-leucyl-O-methyl-L-tyrosine N-methylamide (30 g) and N-methyl morpholine (9.4 g) in acetonitrile (250 ml) was stirred and heated under reflux overnight. A further portion of the amine (1.1 g) was then added and the solution was heated under reflux for a further 4 h. The reaction mixture was then concentrated *in vacuo*, dissolved in chloroform and the solution washed with saturated aq. sodium bicarbonate solution. The material isolated from the organic layer was chromatographed on silica with ethyl acetate as eluant to yield.

N-[3-N-(Benzyloxycarbonyl)amino-1-(R)-methoxycarbonylpropyl]-L-leucyl-O-methyl-L-tyrosine N-methylamide (11.7 g);

(Found: C, 63.09; H, 7.46; N, 9.59. $C_{30}H_{42}N_4O_7$ requires C, 63.16; H, 7.37; N. 9.83%);

$v_{max}$ ($CHCl_3$) 3400, 1720 and 1660 $cm^{-1}$;

σ ($CDCl_3$) 0.86 (6H, m, $CH(CH_3)_2$; 1.2—2.1 (6H, m, $NHCH_2CH_2CH$, $CH_2CH(CH_3)_2$, NH); 2.77 (3H, d, J=5Hz, $NCH_3$); 2.95—3.45 (5H, m, $NHCH_2$, $CH_2C_6H_4$, α*CH*); 3.66 and 3.76 (each 3H, each s, 2 × $OCH_3$); 3.8 and 4.61 (each 1H, each m, 2 × *CH*); 5.10 (2H, m, $CH_2C_6H_5$); 5.21 (1H, m, OCONH); 6.72 (1H, m, CONH); 6.81 (2H, d, J=8.6Hz, Tyr H-3 and H-5); 7.12 (2H, d, J=8.6Hz, Tyr H-2 and H-6); 7.35 (5H, s, $C_6H_5$); 7.55 (1H, d, J=8Hz, CONH); m/e 571 (100§ $[m+1]^+$).

The methyl 4-N-(benzyloxycarbonyl)amino-2-bromobutanoate required in this preparation was made from L-glutamic acid as described in the following paragraphs:

L-Glutamic acid (105 g, 0.713 mol) was dissolved in concentrated sulphuric acid (300 ml) and to this was added chloroform (300ml). To the stirred bi-phasic mixture at 0° was added portionwise over 30 min. sodium azide (60 g, 0.9 mol). The reaction mixture was stirred at 5—10° for 30 min. and was then allowed to slowly warm to room temperature. The reaction mixture was then slowly heated to 80° for one hour the reaction was cooled, poured into water (1.5 1) and the aqueous layer was separated. The aqueous extract was diluted (to 20 litres) and was then applied to Dowex® 50WX8, 16—40 mesh, $H^+$ resin. The column was washed with water and then with 1:1 880 Ammonia/Water and the fractions containing the product were lyophilised.

The crude product obtained above was dissolved in water (1 litre) and to this was added basic copper carbonate (100 g). The stirred mixture was heated under reflux for 40 min. and the hot solution was filtered. The solution was cooled to 35° and $NaHCO_3$ (60 g) and $CHCl_3$ (300 ml) were added. After stirring for 30 min. at room temperature, benzyloxycarbonyl chloride (75 ml) was added and the mixture was then allowed to stir at room temperature overnight. A further portion of benzyloxycarbonyl chloride (30 ml) was then added and stirring was continued for a further 24 h. The crystalline copper complex which had precipitated was then filtered, washed with water and added to a solution of EDTA (di Na salt) (120 g) in water (1.5 litre). The resulting mixture was stirred and heated under reflux for 3 h and was then cooled to 5°. After 40 h at 5° the crystalline product was collected by filtration, washed with water and acetone and dried *in vacuo* at 45°.

The 4-Z-diamino-butyric acid from above (120 g) was suspended in a mixture of dilute sulphuric acid (1M, 600 ml), water (200 ml) and potassium bromide (240 g). Sufficient water (200 ml) was then added to form a single phase. To the resulting solution stirred at −7 to −9°, was added a solution of sodium nitrite (44 g) in $H_2O$ dropwise over 1 h. After 30 min at −7°, the mixture was warmed to room temperature over 1 h. Diethyl ether (1.5 litres) was added and the separated aqueous layer was washed with a further portion of ether. The dried etheral extracts were concentrated *in vacuo* and the residue in methanol (1 litre) was cooled to 0° and treated dropwise with thionyl chloride (65 ml). The reaction was then concentrated *in vacuo* and the residue was partitioned between diethyl ether and saturated aq. sodium bicarbonate. The material isolated from the ether was chromatographed on silica eluting with a gradient of ethyl acetate in hexane to give methyl 4-N-(benzyloxycarbonyl)amino-2-bromo-butanoate (90 g) as an oil which crystallised on standing, m.p. 46—50°; (Found: C, 47.17; H, 5.01; N, 4.16. $C_{13}H_{16}BrNO_4$ requires C, 47.29; H, 4.88; N, 4.24%); δ($CDCl_3$) 2.08—2.45 (2H, m, $CH_2$); 3.37 (2H, m, $NHCH_2$); 3.76 (3H, s, $OCH_3$); 4.32 (1H, dd, J=10Hz and 6Hz, *CH*); 4.97 (1H, broad s, OCONH); 5.09 (2H, s, $OCH_2$) and 7.34 (5H, s, $C_6H_5$).

(b) N-[3-N-(Benzyloxycarbonyl)amino-1-(R)-carboxypropyl]-L-leucyl-O-methyl-L-tyrosine N-Methylamide

To a solution of the preceeding ester (171 mg, 0.3 mmol) in methanol (10 ml) stirred at 0° was added dilute NaOH (1N, 0.6 ml). After stirring overnight at 0° a further portion of NaOH (1N, 0.3 ml) was added and the solution was then stirred for 6h at room temperature. The reaction mixture was then acidified with acetic acid and concentrated to a solid *in vacuo*. Recrystallisation of this material from methanol/$H_2O$ gave the *title compound* (150 mg); m.p. 170—172°; (Found: C, 60.97; H, 7.11; N, 9.68. $C_{29}H_{40}N_4O_7$ + 0.8 $H_2O$ requires C, 60.99; H, 7.34; N, 9.81%); $v_{max}$ (Nujol) 3330, 1690 and 1640 $cm^{-1}$; δ($CD_3OD$) 0.88 (6H, dd, J=14 Hz and 7Hz, $CH(CH_3)_2$); 1.2—1.95 (5H, m, $NHCH_2CH_2$, $CH_2CH(CH_3)_2$); 2.69 (3H, s, $NCH_3$); 2.75—3.65 (6H, m, $NHCH_2$, $CH_2C_6H_4$, and α-*CH* × 2); 3.74 (3H, s, $OCH_3$); 4.54 (1H, dd, J=10Hz and 6Hz, α-*CH*); 5.08 (2H, m, $CH_2C_6H_5$); 6.82 (2H, d, J=8.6Hz, Tyr H-3 and H-5); 7.12 (2H, d, J=8.6Hz, Tyr H-2 and H-6); 7.35 (5H, m, $C_6H_5$).

Example 2

(a) N-[3-N-(Benzyloxycarbonyl)amino-1-(R)-carboxypropyl]-L-leucyl-L-tyrosine N-Methylamide

To a stirred solution of the ester from example 1 (0.35 g, 0.63 mmol) in methanol (10 ml) was added dilute sodium hydroxide (1.3 ml, 1M). After stirring for 42 h at room temperature the reaction mixture was neutralised with acetic acid, concentrated *in vacuo* and partitioned between ethyl acetate and water to give the *title compound* as a solid (121 mg); m.p. 179—182°; (Found: $[m+1]^+$ = 557.2985. $C_{28}H_{38}N_4O_7$ requires 557.2975); δ($d^6$DMSO) 0.8 (6H, m, $CH(CH_3)_2$); 1.18 (2H, m, $CH_2CH(CH_3)_2$); 1.46—1.9 (3H, m, $NHCH_2CH_2$,

$CH_2CH(CH_3)_2$); 2.55 (3H, d, J=5Hz, $CONHCH_3$); 2.6—3.8 (6H, m, $NHCH_2CH_2CHCO_2H$, α-CH, $CH_2$Tyr); 4.38 (1H, dd, J=7 and 15Hz, α-CH); 5.02 (2H, s, $CH_2C_6H_5$); 6.63 (2H, d, J=8Hz, Tyr); 6.97 (2H, d, J=8Hz, Tyr); 7.28 (1H, br, CONH); 7.36 (5H, s, $C_6H_5$); 7.84 (1H, br, CONH); 8.14 (1H, d, J=12Hz, CONH).

(b) N-[3-N-(Benzyloxycarbonyl)amino-1-(R)-methoxycarbonylpropyl]-L-leucyl-L-tyrosine N-Methylamide

To a cold (0°) stirred solution of N-[3-N-(Benzyloxycarbonyl)amino-1-(R)-methoxycarbonylpropyl]-L-leucine (422 mg, 1.01 mmol) and N-methylmorpholine (204 mg) in dichloromethane (10 ml) was added l-hydroxybenzotriazole (154 mg, 1.01 mmol) and N-ethyl-N'-(3-dimethylaminopropyl)carbodiimide hydrochloride (194 mg, 1.01 mmol). After 15 minutes at 0° a solution of L-tyrosine N-methylamide hydrochloride (235 mg, 1.01 mmol) in dichloromethane/dimethylformamide (11 ml, 10:1) was added followed by N-methylmorpholine (102 mg, 1.01 mmol). The reaction mixture was then allowed to warm and stir to room temperature overnight. The reaction mixture after washing with water, 3N citric acid, saturated aqueous sodium bicarbonate solution and water was dried and concentrated *in vacuo* to a foam (0.35 g) δ($CDCl_3$) 0.85 (6H, m, $CH_2(CH_3)_2$); 1.1—2.0 (6H, m, $NHCH_2CH_2$, $CH_2CH(CH_3)_2$); 2.74 (3H, d, J=5Hz, $CONHCH_3$); 2.95—3.4 (6H, m, $NHCH_2CH_2CH$, α-CH, $CH_2C_6H_4$); 3.64 (3H, s, $OCH_3$); 4.62 (1H, dd, J=7 and 15Hz, α-CH); 5.10 (2H, s, $CH_2C_6H_5$); 5.32 (1H, br, CONH); 6.78 (2H, d, J=8Hz, $C_6H_4$); 6.86 (1H, br, CONH); 7.02 (2H, d, J=8Hz, $C_6H_4$); 7.35 (5H, s, $C_6H_5$); 7.65 (1H, d, J=10Hz, CONH).

L-tyrosine N-methylamide hydrochloride used in this preparation was prepared as follows:

(c) L tyrosine N-Methylamide hydrochloride

To a solution of O-benzyl-L-tyrosine N-methylamide HCl (1.13 g) in methanol (50 ml) was added 10% Pd/C and the mixture was stirred in an atmosphere of hydrogen at room temperature and atmospheric pressure for 4 h. The catalyst was then filtered and the solvent was removed *in vacuo* to yield L-tyrosine N-methylamide HCl as a foam (0.85 g).

(d) N-[3-N-(Benzyloxycarbonyl)amino-1-(R)-methoxycarbonylpropyl]-L-leucine

N-[3-N-(Benzyloxycarbonyl)amino-1-(R)-methoxycarbonylpropyl]-L-leucine t-butyl ester (4.2 g) was treated with Trifluoroacetic acid (50 ml) and water (5 ml) for one hour at room temperature. Volatiles were then removed *in vacuo* and the residue was co-evaporated with ethereal HCl. Recrystallisation of the residue from methanol/ether gave the title compound as its hydrochloride salt (2.2 g) m.p. 103—109°; (Found: C, 54,55; H, 7.37; N, 6.37. $C_{19}H_{29}N_2O_6Cl$ requires C, 54.74; H, 7.01; N, 6.72%); δ($d^6$DMSO) 0.9 (6H, m, $CH(CH_3)_2$); 1.72 (3H, m, $CH_2CH(CH_3)_2$); 2.05 (2H, m, $NHCH_2CH_2$); 3.12 (2H, m, $NHCH_2$); 3.74 (3H, s, $OCH_3$); 3.82 (1H, m, α-CH); 4.02 (1H, m, α-CH); 5.02 (2H, s, $CH_2C_6H_5$); 7.35 (5H, br, s, $C_6H_5$).

The foregoing t-Butyl ester was prepared as described below:

(e) N-[3-N-(Benzyloxycarbonyl)amino-1-(R)-methoxycarbonylpropyl]-L-leucine t-Butyl ester

To a solution of methyl 4-N-(benzyloxycarbonyl)amino-2-bromo-butanoate (64 g) and L-leucine t-butyl ester (34 g) in acetonitrile (300 ml) was added N-methylmorpholine (20 g) and the solution was heated under reflux for 48 h. The reaction mixture was then cooled, concentrated *in vacuo*, taken up in water and extracted with ether. The combined ethereal extracts were then dried and concentrated *in vacuo* to an oil. Column chromatography of this oil on silica eluting with a gradient of ethyl acetate in hexane gave the *title compound* as an oil (20 g); δ($CDCl_3$) 0.87 (6H, m, $CH(CH_3)_2$); 1.44 (9H, s, $OC(CH_3)_3$); 1.4—1.95 (5H, m, $NHCH_2CH_2$, $CH_2CH(CH_3)_2$); 3.05—3.5 (4H, m, $NHCH_2$, α-CH × 2); 3.70 (3H, s, $OCH_3$); 5.10 (2H, s, $CH_2C_6H_5$); 5.73 (1H, m, CONH); 7.36 (5H, br, s, $C_6H_5$).

Example 3

(a) N-(3-N-(Benzyloxycarbonyl)amino-1-(R)-carboxypropyl-L-leucyl-O-propyl-L-tyrosine N-methyl amide

(a) N-(3-N-(Benzyloxycarbonyl)amino-1-(R)-methoxycarbonylpropyl)-L-leucyl-O-propyl-L-tyrosine N-methyl amide (210 mg, 0.35 mmol) in methanol (10 ml) was treated with aqueous sodium hydroxide (1.1 ml, 0.5 M) at room temperature for 48 h. The reaction was then neutralised with acetic acid and concentrated *in vacuo* to afford N-(3-N-Benzyloxycarbonyl)amino-1-(R)-carboxypropyl)-L-leucyl-O-propyl-L-tyrosine N-methyl amide which crystallised from methanol/water as needles m.p. 160—167°C (Found: C, 63.2; H, 7.8; N, 9.7. $C_{31}H_{44}N_4O_7$ + 0.3 $H_2O$ requires: C, 63.7; H, 7.6; N, 9.6%); δ ($CDCl_3$) 0.8 (9H, m $(CH_3)_2CH$, $CH_3CH_2$); 0.88—1.88 (9H, m, CH, $CH_2$, $CH_2$, $CH_2CH_2$); 2.58 (3H, d, J=4Hz, $CH_3NH$); 2.2—3.66 (5H, m, $CH_2$, CH, $CH_2$); 3.84 (2H, t, J=6Hz, $CH_2$—O); 4.4 (1H, m, NHCHCO); 5.02 (2H, s, O—$CH_2C_6H_5$); 5.6 (1H, m, NH); 6.76, and 7.08 (4H, each d, each J=7Hz, $C_6H_4$); 7.24 (1H, m, NH); 7.32 (5H, m, $C_6H_5$); 7.8 (1H, m, NH); 8.14 (1H, m, NH).

The preceeding methyl ester was prepared as follows:

(b) N-(3-N-(Benzyloxycarbonyl)amino-1-(R)-methoxycarbonylpropyl)-L-leucyl-O-propyl-L-tyrosine N-methyl amide

N-Tertiary butyloxycarbonyl-O-propyl-L-tyrosine N-methyl amide (370 mg, 1.1 mmol) in dichloromethane (8 ml) was treated with trifuoroacetic acid (2 ml) at 20°C for 2 h. The solvent was removed *in vacuo* and the residue redissolved in ether saturated with hydrogen chloride and this procedure repeated twice more to afford O-propyl-L-tyrosine-N-methyl amide hydrochloride (1.1 mM) which was used directly

in the next step. N-(3-N-(Benzyloxycarbonyl)amino-1-(R)-methoxycarbonylpropyl)-L-leucine hydrochloride (prepared as described in Example 1) (416 mg, 1 mmol) in dichloromethane (10 ml) and dimethyl formamide (2 ml) was treated with 1-hydroxybenzotriazole (159 mg, 1.04 mmol), O-propyl-L-tyrosine-N-methyl amide hydrochloride (1.1 mmol), N-methylmorpholine (315 mg, 3.3 mmol) and N-ethyl-N'-(3-dimethylaminopropyl)-carbodiimide hydrochloride (200 mg, 1.04 mmol) at 0°C. The reaction mixture was stirred continously as it was allowed to warm to 20°C over 16 h. The reaction was diluted with dichloromethane (30 ml) and washed successively with water, aqueous saturated sodium hydrogen carbonate and aqueous citric acid (1 M) dried over anhydrous sodium sulphate and concentrated *in vacuo* to afford N-(3-N-(Benzyloxycarbonyl)amino-1-(R)-methoxycarbonylpropyl)-L-leucyl-O-propyl-L-tyrosine N-methyl amide (0.22g, 0.36 mmol) which crystallised from ethyl acetate/hexane as needles m.p. 135—140°C (Found: C, 62.8; H, 7.8; N, 9.2. $C_{32}H_{46}N_4O_7$ + 0.5 $H_2O$ requires: C, 63.2; H, 7.8; N, 9.2%); δ($CDCl_3$) 0.86 (6H, d, d, J=4Hz J=4Hz, $(CH_3)_2C$); 1.02 (3H, t, J=7Hz, $CH_3CH_2$); 1.1—1.98 (9H, m, CH, $CH_2$, $CH_2$, $CH_2CH_2$); 2.74 (3H, d, J=4Hz, $CH_3NH$); 2.86—3.48, (5H, m, $CH_2CH_2$, CH); 3.64 (3H, s, $OCH_3$); 3.86 (2H, t, J=6Hz, $CH_2O$); 4.58 (1H, m, NHC*H*CO); 5.06 (2H, s, $CH_2C_6H_5$); 5.14 (1H, m, NH) 6.54(1H, m, NH); 6.76, and 7.06 (4H, each d, each J=8Hz, $C_6H_4$); 7.32 (5H, m, $C_6H_5$); 7.42 (1H, m, NH).

The intermediate used in the preceeding section was prepared as follows:

(c) N-Tertiarybutyloxycarbonyl-O-propyl-L-tyrosine N-methyl amide

Tertiarybutyloxycarbonyl-L-tyrosine (14.1 g, 50 mmol) in dimethyl formamide (200 ml) was cooled to 10°C and treated with sodium hydride (80%, 3.3 g. 110 mmol) with continuous stirring for 1 h. Propyl bromide (6.15 g, 50 mmol) was added and the reaction allowed to warm to 20°C over 16h. Water (50 ml) was added and the reaction concentrated *in vacuo* to 100 ml, water 300 ml was added and the solution washed twice with ethyl acetate (300 ml). The reaction was then acidified to pH 1 with hydrochloric acid (6N) and extracted twice with ethyl acetate. The organic extract was dried over anhydrous sodium sulphate and concentrated *in vacuo* to a gum. Column chromatography on silica in ethyl acetate afforded N-tertiarybutyl oxycarbonyl-O-propyl-L-tyrosine (15.1 g 46.7 mmol) as a foam which was used directly in the next step. To (13 g, 40 mmol) of this foam in dichloromethane (250 ml) was added 1-hydroxybenzotriazole (6.8 g, 45 mmol); methylamine hydrochloride (2.5 g, 80 mmol); N-methyl morpholine (8 g, 80 mmol) and dicyclohexylcarbodimide (9.1 g, 45 mmol) at 0°C. The reaction was stirred continuously whilst allowed to warm to 20°C over 6h. The reaction was filtered and the filtrate washed with water, aqueous saturated sodium hydrogen carbonate and aqueous citric acid (1M), dried over anhydrous sodium sulphate and concentrated *in vacuo* to afford N-tertiarybutyloxycarbonyl-O-propyl-L-tyrosine-N-methyl-amide (8.5 g, 25 mmol) which crystallised from ethyl acetate as needles m.p. 134—135° (Found: C, 64.5; H, 8.7; N, 8.4. $C_{18}H_{28}N_2O_4$ requires: C, 64.3; H, 8.4; N, 8.3%; δ($CDCl_3$) 0.96 (3H, t, J=8Hz, $CH_3CH_2$); 1.36 (9H, s, $(CH_3)_3C$); 1.74 (2H, t, q, J=7Hz, J=7Hz, $CH_2$); 2.68 (3H, d, J=4Hz, $CH_3NH$); 2.92 (2H, m, $CH_2C_6H_4$); 3.82 (2H, t, J=7Hz, $CH_2$—O); 4.16 (1H, m, NHC*H*CO); 4.98 (1H, m, NH); 5.68 (1H, m, NH); 6.74 and 7.0 (4H, each 2H, each d, each J=8Hz, $C_6H_4$).

Example 4

(a) N-[3-N-(Benzyloxycarbonyl)amino-1-(R)-carbonypropyl]-L-leucyl-O-isopropyl-L-tyrosine N-methylamide

N-[3-N-(Benzyloxycarbonyl)amino-1-(R)-methoxycarbonypropyl]-L-leucyl-O-isopropyl-L-tyrosine N-methylamide (0.4 g, 0.65 mmol) in methanol (15 ml) was treated with aqueous sodium hydroxide (2 ml, 0.5 M) at 20°C for 24 h. The solution was then adjusted to pH7 with acetic acid and concentrated to low volume. The white solid which crystallised was recrystallised from methanol/water 1:1 to afford N-[3-N-(Benzyloxycarbonyl)amino-1-(R)-carboxypropyl]-L-leucyl-O-isopropyl-L-tyrosine-N-methyl amide (100 mg, 0.17 mmol) m.p. 148—160°C (Found: C, 63.3; H, 7.6; N, 9.6. $C_{31}H_{44}N_4O$ requires: C, 63.7; N, 7.8; N, 9.3%) δ($CDCl_3$) 0.8 (6H, m, $(CH_3)_2C$); 0.98—1.84 (5H, m, $CH_2$, $CH_2$, CH); 1.22 (6H, d, J=6Hz, $(CH_3)_2CHO$); 2.56 (3H, d, J=4Hz, $CH_3NH$); 2.64—3.66 (7H, m, $CH_2$, $CH_2$, $CH_2$, CH); 4.4 (1H, m, NHC*H*CO); 4.52 (1H, m, NHC*H*CO); 5.0 (2H, s, $OCH_2C_6H_5$); 6.72, and 7.06 (each 2H, each d, each J=8Hz $C_6H_4$); 7.32 (5H, m, $C_6H_5$); 7.88 (1H, m, NH); 8.22 (1H, m, NH).

The preceeding methyl ester was prepared as follows:

(b) N-[3-N-(Benzyloxycarbonyl)amino-1-(R)-methoxycarbonylpropyl]-L-leucyl-O-isopropyl-L-tyrosine N-methylamide

N-Tertiary butyloxycarbonyl-O-isopropyl-L-tyrosine-N-methylamide (370 mg, 1.1 mmol); in dichloromethane (10 ml) was treated with trifluoroacetic acid (10 ml) at 20°C for 1 h. The resulting trifluoroacetate was converted to the hydrochloride salt by concentrating to a gum *in vacuo* and redissolving the residue in ether saturated with hydrogen chloride. This procedure was repeated three times. To the product in dichloromethane (10 ml) was added N-[3-N-(Benzyloxycarbonyl)amino-1-(R)-methoxycarbonylpropyl]-L-leucine hydrochloride (416 mg, 1 mmol) (prepared as described in Example 1) 1-hydroxybenzotriazole (159 mg, 10.4 mmol), and N-(3-dimethylaminopropyl)-N'-ethylcarbodiimide hydrochloride (200 mg, 1.04 mmol) at 0°C. The stirred reaction mixture was adjusted to pH7 with N-methylmorpholine and allowed to warm to room temperature over 16 h. The reaction was diluted with dichloromethane and washed with water, aqueous saturated sodium hydrogen carbonate, and aqueous citric acid (1M), dried over sodium sulphate and concentrated *in vacuo* to a gum. (Found: $[M+H]^+$ =

599.3446. $C_{32}H_{46}N_4O_7$ requires: $[M+H]^+$ = 599.3445); δ($CDCl_3$) 0.84 (6H, m, $(CH_3)_2CH$) 1.3 (6H, d, J=7Hz, $(CH_3)_2CHO$) 1.1—1.96 (5H, m, 2 × $CH_2$, CH) 2.72 (3H, d, J=4Hz, $CH_3NH$); 3.04—3.36 (6H, m, 2 × $CH_2$, 2 × CH); 3.64 (3H, s, $CH_3O$); 4.46 (1H, m, NHC*H*CO); 4.56 (1H, m, NHC*H*CO); 5.06 (2H, s, O—($CH_2C_5H_6$); 5.32 (1H, m, NH); 6.76, and 7.04 (each 2H, each d, each J=8Hz, $C_6H_4$); 6.98 (1H, m, NH); 7.3 (5H, m, $C_6H_5$); 7.52 (1H, m, NH).

The intermediate used in the preceeding section was prepared as follows:

(c) N-Tertiarybutyloxycarbonyl-O-isopropyl-L-tyrosine N-methylamide

N-Tertiarybutyloxycarbonyl-L-tyrosine (14.1 g, 50 mmol) in dry dimethylformamide (200 ml) was treated with sodium hydride (3.45 g, 115 mmol) with vigorous stirring under an atmosphere of argon at 10°C for 1 h. 2-Bromopropane (6.15 g, 50 mmol) was added and the stirred solution allowed to warm to 20°C over 16 h. Water (200 ml) was added and the solution extracted with ethyl acetate (2 × 200 ml). The reaction was adjusted to pH 1 with hydrochloric acid (6M) and extracted with dichloromethane 2 × 300 ml. The combined extracts were columned on silica in ethyl acetate to afford N-tertiarybutyloxycarbonyl-O-isopropyl-L-tyrosine (13.6 g, 42 mmol) as a foam. The foam was dissolved in dichloromethane (200 ml) and treated with methylamine hydrochloride (2.5 g 80 mmol), 1-hydroxybenzotriazole (6.75 g, 44 mM), dicyclohexylcarbodiimide (9.1 g, 44 mmol) and N-methyl morpholine (2.5 g, 80 mmol) at 0°C with continuous stirring. The solution was allowed to warm to room temperature over 16 h and filtered. The filtrate was washed with water, aqueous saturated sodium hydrogen carbonate solution and aqueous citric acid (1 M), dried over sodium sulphate and concentrated *in vacuo* to afford N-tertiarybutyloxycarbonyl-O-isopropyl-L-tyrosine-N-methyl amide (6.1 g, 18 mmol) which crystallised from ethyl acetate/hexane as needles m.p. 110—114°C (Found: C, 63.5; H, 8.4; N, 8.7. $C_{18}H_{28}N_2O_4$ 0.25 $H_2O$ requires: C, 63.4; H, 8.4; N, 8.2%) δ($CDCl_3$) 1.32 (6H, d, J=7Hz, $(CH_3)_2CH$); 1.4 (9H, s, $(CH_3)_3C$); 2.72 (3H, d, J=4Hz, $NHCH_3$); 2.98 (2H, d, J=7Hz, $CH_2C_6H_5$); 4.26 (1H, m, NHCHCO); 4.5 (1H, heptet, J=5Hz, C*H*$(CH_3)_2$); 5.08 (1H, m, NH); 5.92 (1H, m, NH); 6.78, and 7.04 (each 2H, each d, each J=8Hz $C_6H_4$).

Example 5

(a) N-[3-N-(Benzyloxycarboxyl)(amino-1-(R)-carboxypropyl)]-L-leucyl-O-tertiarybutyl-L-tyrosine. N-methyl amide

N-[3-N-(Benzyloxycarbonyl)amino-1-(R)-methoxycarbonylpropyl]-L-leucyl-O-tertiary butyl-L-tyrosine N-methyl amide (0.26 g, 0.42 mmol) in methanol (10 ml) was treated with aqueous sodium hydroxide (1.3 ml, 0.5 M) at 20°C for 24 h. The reaction was then adjusted to pH 7 with hydrochloric acid (6N) and concentrated *in vacuo* to low volume. The white solid which crystallized out was washed with ether and water and dried *in vacuo* to afford N-[3-N-(Benzyloxycarbonyl)amino-1-(R)-carboxypropyl-L-leucyl-O-tertiary butyl-L-tyrosine-N-methyl amide m.p. 160°C dec. (Found: C, 62.51; H, 7.67; N, 9.24; $C_{32}H_{46}N_4O_7 \cdot H_2O$ requires: C, 62.32; H, 7.84; N, 9.08%). δ($CDCl_3$) 0.78 (6H, m $(CH_3)_2C$); 1.08—1.86 (5H, m, $CH_2$, $CH_2CH$); 1.24 (9H, s, $(CH_3)_3C$); 2.56 (3H, d, J=4Hz, $CH_3NH$); 2.44—3.2 (6H, m, 2 × $CH_2$, 2 × CH); 4.4 (1H, m, NHC*H*CO); 5.0 (2H, s, $CH_2C_5H_6$); 6.82 and 7.08 (each 2H, each d, each J=8Hz, $C_6H_4$); 7.22 (1H, m, NH); 7.3 (5H, m, $C_6H_5$); 7.84 (1H, m, NH); and 8.2 (1H, m, NH).

The preceeding methyl ester was prepared as follows:

(b) N-[3-N-(Benzyloxycarbonyl)amino-1-(R)-methoxycarbonylpropyl]-L-leucyl-O-tertiary butyl-L-tyrosine N-methyl amide

N-Benzyloxycarbonyl-O-tertiarybutyl-L-tyrosine N-methylamide (2.4 g, 6.2 mmol) in ethanol (10 ml) was heated under reflux with cyclohexene (4 ml) acetic acid (0.38 g, 6.2 mmol) and palladium on carbon (10%, 1g) under an atmosphere of argon for 20 min. The solvents were then removed *in vacuo* to afford O-tertiarybutyl-L-tyrosine N-methylamide acetate m.p. 121—123°C. This was dissolved in dichloromethane (50 ml) and shaken with aqueous saturated sodium hydrogen carbonate (50 ml). The organic layer was dried over anhydrous sodium sulphate and concentrated *in vacuo* to afford O-tertiary butyl-L-tyrosine N-methyl amide as a crude gum which was used directly in the next step. N-[3-N-(Benzyloxycarbonyl)amino-1-(R)-methoxycarbonylpropyl]-L-leucine hydrochloride (418 mg, 1 mmol) in dichloromethane (10 ml) was treated with 1-hydroxybenzotriazole (159 mg, 1.0 mmol) N-methylmorpholine (210 mg, 2 mmol), O-tertiarybutyl-L-tyrosine-N-methyl amide (275 mg, 1.1 mmol) and N-(3-dimethylaminopropyl)-N′-ethyl carbodiimide hydrochloride (200 mg, 1.04 mol) at 0°C with continuous stirring. The reaction mixture was adjusted to pH 7 with N-methylmorpholine and was allowed to warm to 20°C over 16 h. The reaction was diluted with dichloromethane and washed successively with water, aqueous saturated sodium hydrogen carbonate and aqueous 3N citric acid, dried over sodium sulphate and concentrated *in vacuo* to afford N-[3-N-(Benzyloxycarbonyl)amino-1-(R)-methoxycarbonypropyl]-L-leucine-O-tertiary butyl-L-tyrosine N-methyl amide (350 mg, 0.6 mmol) whch crystallised from ethyl acetate/hexane as needles m.p. 66—68°C (Found: C, 64.7; H, 8.1; N, 9.2. $C_{33}H_{48}N_4O_7$ requires: C, 4.7; H, 7.9; N, 9.1%); δ($CDCl_3$) 0.88 (6H, d, J=7Hz, $(CH_3)_2C$); 1.32 (9H, s, $(CH_3)_3C$); 1.16—2.0 (5H, m, $CH_2$, $CH_2CH$); 2.72 (3H, d, J=5Hz, $CH_3NH$); 3.02 to 3.4 (6H, m, 2 × CH, 2 × $CH_2$); 3.66 (3H, s, $CH_3O$); 4.58 (1H, m, NHC*H*CO); 5.08 (2H, s, $CH_2C_5H_6$); 5.2 (1H, m, NH); 6.54 (1H, m, NH); 6.88 and 7.06 (each 2H, each d, each J=8Hz, $C_6H_4$); 7.32 (5H, m, $C_6H_5$); 7.52 (1H, m, NH).

The foregoing intermediate was prepared as follows:

(c) N-Benzyloxycarbonyl-O-tertiarybuty-L-tyrosine N-methyl amide

Benzyloxycarbonyl-O-tertiarybutyl-L-tyrosine dicyclohexyclamine salt (5 g, 0.9 mmol) was dissolved in

aqueous citric acid (25 ml, 1M) and extracted with ethyl acetate (3 × 50 ml). The organic layer was dried over sodium sulphate and concentrated *in vacuo* to afford the free acid (4.1 g) as a gum. This was dissolved in dichloromethane (100 ml) and treated with hydroxy benzotriazole (1.5 g, 10 mM), methylamine hydrochloride (0.56 g, 18 mmol) and dicyclohexyl carbodiimide (2.1 g, 10 mmol) at 0°C with continuous stirring. The reaction was allowed to warm to 20°C over 16 h and then filtered. The filtrate was diluted with dichloromethane and washed with water aqueous saturated sodium hydrogen carbonate and aqueous citric acid (1M), dried over sodium sulphate and concentrated *in vacuo* to afford Benzyloxycarbonyl-O-tertiary butyl-L-tyrosine N-methylamide (2.5 g) which crystallised from ethyl acetate/hexane as needles m.p. 124—125°C (Found: C, 68.7; H, 7.9: N, 7.4. $C_{22}H_{28}N_2O_4$ requires: C, 68.7; H, 7.3; N, 7.3%); δ($CDCl_3$) 1.34 (9H, s, $(CH_3)_3C$); 2.68 (3H, d, J=4Hz, NHC*H*$_3$); 2.94 and 3.1 (2H, each, dd, each J=15Hz, J=6Hz, C*H*$_2$$C_6H_4$); 4.3 (1H, q, J=5Hz, NHC*H*CO); 5.08, (2*H*, s, C*H*$_2$$C_6H_5$); 5.34 (1H, m, NH); 5.58 each (1H, m, NH); 6.88 and 7.06 (each 2H, each d, each J=8Hz, $C_6H_4$); 7.3 (5H, m, $C_6H_5$).

Example 6

(a) N-[3-N-(Benzyloxycarbonyl)amino-1-(R)-carboxypropyl]-L-leucyl-O-pentyl-L-tyrosine N-Methylamide

To a stirred solution of 1 (280 mg, 0.47 mmol) in methanol (10 ml) was added dilute sodium hydroxide (0.7 ml, 1M). After stirring for 48 h at room temperature the reaction was neutralised with acetic acid and was concentrated *in vacuo* to a solid. Recrystallisation from methanol/water gave the *title compound* as a solid (107 mg); m.p. 165—170°C; δ ($d^6$DMSO) 0.8 (6H, dd, J=5Hz, J=$CH_2CH$(C*H*$_3$)$_2$); 0.87 (3H, t, J=7Hz, $CH_2$C*H*$_3$); 1.1—1.25 (2H, m, C*H*$_2$CH$(CH_3)_2$); 1.26—1.85 (9H, m, $OCH_2$(C*H*$_2$)$_3$$CH_3$), $NHCH_2$C*H*$_2$CH, $CH_2$C*H*$(CH_3)$); 2.56 (3H, d, J=8Hz, CONHC*H*$_3$); 2.6—3.65 (6H, m, NHC*H*$_2$$CH_2$C*H*$CO_2H$, α-CH, $CH_2$Tyr); 3.87 (2H, t, J=7Hz, OC*H*$_2$$CH_2$); 4.42 (1H, dd, J=7 and 15Hz, α-CH); 5.02 (2H, s, C*H*$_2$$C_6H_5$); 6.77 (2H, d, J=8Hz, Tyr); 7.10 (2H, d, J=8Hz, Tyr); 7.26 (1H, br, CONH); 7.35 (5H, s, $C_6H_5$); 7.85 (1H, br, CONH); 8.14 (1H, d, J=8Hz, CONH).

The preceeding ester was synthesised as described below:

(b) N-[3-N-(Benzyloxycarbonyl)amino-1-(R)-methoxycarbonylpropyl]-L-leucyl-O-pentyl-L-tyrosine N-Methylamide

To a solution of N-[3-N-(Benzyloxycarbonyl)amino-1-(R)-methoxycarboxypropyl]-L-leucine hydrochloride (418 mg, 1 mmol) in dichloromethane (10 ml), stirred and cooled to 0° was added N-methylmorpholine (101 mg), 1-hydroxybenzotriazole (153 mg, 1 mmol) and dicyclohexylcarbodiimide (206 mg, 1 mmol). After 15 minutes at 0° a solution of O-pentyl-L-tyrosine N-methylamide (265 mg) (which had been prepared from the N-benzyloxycarbonyl precursor in the usual manner) was added dissolved in dichloromethane (10 ml). The reaction after being allowed to warm and stir to room temperature overnight was filtered, washed with water, 3N citric acid, saturated aqueous sodium bicarbonate and water. The washed organic extract was dried and concentrated to an oil *in vacuo*. Chromatography on silica eluting with ethyl acetate gave N-[3-N-(benzyloxycarbonyl)amino-1-(R)-methoxycarbonylpropyl]-L-leucyl-O-pentyl-L-tyrosine N-Methylamide as a solid (359 mg); m.p. 81—85°; (Found: C, 64.78; H, 8.05; N, 8.82. $C_{34}H_{52}N_4O_7$ requires C, 64.94; H, 8.34; N, 8.91%); δ ($CDCl_3$) 0.83 (3H, d, J=5Hz, $CH_2CH$(C*H*$_3$)); 0.87 (3H, d, J=5Hz, $CH_2CH$(C*H*$_3$)); 0.92 (3H, d, J=7Hz, $CH_2$C*H*$_3$); 1.0—2.0 (12H, $NHCH_2$C*H*$_2$, NH, C*H*$_2$C*H*$(CH_3)_2$, $OCH_2$(C*H*$_2$)$_3$$CH_3$); 2.75 (3H, d, J=5Hz, CONHC*H*$_3$); 2.9—3.6 (6H, m, NHC*H*$_2$$CH_2$C*H*$CO_2H$, α-CH, $CH_2$Tyr); 3.66 (3H, s, $OCH_3$); 3.9 (2H, t, J=7Hz, OC*H*$_2$$CH_2$); 4.58 (1H, dd, J=7 and 15Hz, CH); 5.11 (3H, br, C*H*$_2$$C_6H_5$, CONH); 6.58 (1H, br, CONH); 6.81 (2H, d, J=8Hz, $C_6H_4$); 7.10 (2H, d, J=8Hz, $C_6H_4$); 7.36 (5H, s, $C_6H_5$); 7.47 (1H, d, J=8Hz, CONH).

N-Benzyloxycarbonyl-O-pentyl-L-tyrosine N-methylamide used in this preparation was synthesised as follows:

(c) N-(Benzyloxycarbonyl)-O-pentyl-L-tyrosine N-Methylamide

To a cold (0°) stirred solution of N-benzyloxycarbonyl-O-pentyl-L-Tyrosine (8.3 g, 22 mmol) in THF (120 ml) was added 1-hydroxybenzotriazole (3.6 g, 24 mmol) and dicyclohexylcarbodiimide (4.9 g, 24 mmol). After stirring for 25 minutes at 0° a solution of methylamine in THF (7.2 ml, 5M) was added and the reaction was stirred and warmed to room temperature overnight. The reaction mixture was then filtered, evaporated *in vacuo* and dissolved in dichloromethane (150 ml). The dichloromethane extract after washing with water, 3N citric acid, saturated sodium bicarbonate solution and water was dried, filtered and concentated *in vacuo* to a solid. Recrystallisation from ethyl acetate gave the required N-Methylamide as needles (6.43 g); m.p. 132—135°; (Found: C, 69.30; H, 7.63; N, 7.43. $C_{23}H_{30}N_2O_4$ requires C, 69.32; H, 7.59; N, 7.03%); δ($CDCl_3$) 0.92 (3H, t, J=7Hz, $CH_2$C*H*$_3$); 1.25—2.0 (6H, m, $OCH_2$(C*H*$_2$)$_3$$CH_3$); 2.71 (3H, t, J=5Hz, CONHC*H*$_3$); 3.0 (2H, m, $CH_2$Tyr); 3.92 (3H, t, J=7Hz, $OCH_2$C*H*$_3$); 4.29 (1H, dd, J=7 and 15Hz, α-CH); 5.09 (2H, s, C*H*$_2$$C_6H_5$); 5.37 (1H, br, CONH); 5.69 (1H, br, CONH); 6.82 (2H, d, J=8Hz, Tyr); 7.08 (2H, d, J=8Hz, Tyr), 7.33 (5H, m, $C_6H_5$).

The preceeding acid was synthesised as described below.

(d) N-(Benzyloxycarbonyl)-O-pentyl-L-tyrosine

To a cold (10°) stirred solution of N-benzyloxycarbonyl-L-tyrosine (12.61 g, 40 mmol) in dry DMF (150 ml) under argon was added sodium hydride (2.76 g, 80% dispersion in oil). After stirring for one hour at 10° n-bromopentane (6.04, 40 mmol) was added and the reaction mixture was allowed to stir and warm to

room temperature overnight. Water (800 ml) and ethyl acetate (800 ml) were then added and the separated organic phase was acidified and extracted with ethyl acetate (200 ml × 3). This organic extract was dried, filtered and concentrated to an oil *in vacuo*. Chromatography on silica eluting with ethyl acetate gave N-benzyloxycarbonyl-O-pentyl-L-Tyrosine which was recrystallised from ether/hexane to give a solid (8.5 g), m.p. 77—81°; (Found: C, 68.58; H, 7.19; N, 3.77. $C_{22}H_{27}NO_5$ requires C, 68.55; H, 7.06; N, 3.63%); δ($CDCl_3$) 0.92 (3H, t, J=7Hz, $CH_2$*$CH_3$*); 1.29—1.52 (4H, m, $CH_2$ alkyl); 1.66—1.86 (2H, m, $CH_2$ alkyl); 3.0—3.12 (2H, m, $CH_2$Tyr); 3.93 (2H, t, J=7Hz, O*$CH_2$*$CH_3$); 4.67 (1H, m, CH); 5.12 (2H, s, *$CH_2$*$C_6H_5$); 5.2 (1H, d, J=8Hz, CONH); 6.82 (2H, d, J=8Hz, $C_6H_4$); 7.07 (2H, d, J=8Hz, $C_6H_4$); 7.35 (5H, s, $C_6H_5$).

Example 7

(a) N-[3-N-(Benzyloxycarbonyl)amino-1-(R)-carboxypropyl]-L-leucyl-O-(3-methylbutyl)-L-tyrosine N-methyl amide

N - [3 - N - (Benzyloxycarbonyl)amino - 1-(R)-methoxy-carbonylpropyl]-L-leucyl-O-(3-methylbutyl)-L-tyrosine N-methyl amide (270 mg, 0.43 mmol) in methanol (10 ml) was treated with aqueous sodium hydroxide (1.3 ml, 0.5 M) at 20°C for 48 h. The reaction was adjusted to pH4 with acetic acid and the solvent removed *in vacuo* to afford N-[3-N-(Benzyloxycarbonyl)amino-1-(R)-carboxypropyl]-L-leucyl-O-(3-methylbutyl)-L-tyrosine N-methylamide (121 mg) which crystallised from methanol/water as needles m.p. 155—160°C (Found: C, 63.7; H, 7.7; N, 9.2. $C_{33}H_{48}N_4O_7$ requires: C, 64.7; H, 7.9; N, 9.1%); δ($(CD_3)_2SO$) 0.8 and 0.92 (12H, each m, 2 × $(CH_3)_2C$); 1.0—1.9 (8H, m, 3 × $CH_2$, 2 × CH) 2.56 (3H, d, J=4Hz, *$CH_3$*NH) 2.76—3.18 (6H, m, 2 × $CH_2$, 2 × CH); 3.92 (2H, m, $CH_2O$); 4.52 (1H, m NHC*H*CO); 5.0 (2H, s, *$CH_2$*$C_6H_5$); 6.76 and 7.1 (each 2H, each d, each J=8Hz, $C_6H_4$); 7.34 (5H, m, $C_6H_5$); 7.9 (1H, m, NH); 8.1 (1H, m, NH).

The preceeding methyl ester was prepared as described below:

(b) N-[3-N-(Benzyloxycarbonyl)amino-1-(R)-methoxycarbonylpropyl]-L-leucyl-O-(3-methylbutyl)-L-tyrosine N-Methyl amide

Tertiarybutyloxycarbonyl-O-(3-methylbutyl)-L-tyrosine-N-methylamide (401 mg, 1.1 mmol) in dichloromethane (10 ml) was treated with trifluoroacetic acid (10 ml) at 20°C for 1. 5 h. The solution was concentrated *in vacuo* to a gum and dissolved in ether saturated with hydrogen chloride, and this procedure repeated twice to afford O-(3-methylbutyl)-L-tyrosine-N-methylamide hydrochloride which was used directly in the next step. N(3-N-(Benzyloxycarbonyl)amino-1-(R)-methoxycarbonylpropyl)-L-leucine Hydrochloride (418 mg 1 mmol) in dichloromethane (20 ml) and dimethyl formamide (2 ml) was treated with N-methyl morpholine (420 mg, 4 mmol), 1-hydroxybenzo- triazole (159 mg 1.04 mmol), O-(3-methylbutyl)-L-tyrosine-N-methyl amide hydrochloride (1.1 mM) and N-ethyl-N'-(3-dimethylaminopropyl)-carbodiimide hydrochloride (200 mg, 1.04 mmol) at 0°C with continuous stirring. The reaction was allowed to warm to 20°C over 16 h. The reaction was diluted with dichloromethane (30 ml) and washed successively with water, aqueous saturated sodium hydrogen carbonate and aqueous citric acid (1M), dried over sodium sulphate and concentrated *in vacuo* to afford N-[3-N-(Benzyloxycarbonyl)amino-1-(R)-methoxy-carbonypropyl]-L-leucyl-O-3(methyl)butyl-L-tyrosine N-methylamide (360 mg, 0.57 mmol) which crystallised from ethyl acetate/hexane as needles m.p. 78—81°C. (Found: C, 65.8; H, 8.0; N, 8.9 $C_{34}H_{50}N_4O_7$ requires: C, 64.8; H, 8.3; N, 8.9); δ($CDCl_3$) 0.86 (6H, dd, J=6Hz, J=1Hz $(CH_3)_2C$) 0.94 (6H, d, J=6Hz, $(CH_3)_2C$); 1.02—2.0 (8H, m, 3 × ($CH_2$), 2 × CH); 2.74 (3H, d, J=4Hz, *$CH_3$*NH); 2.92—3.42 (6H, m, 2 × $CH_2$, 2 × CH); 3.66 (3H, s, $CH_3O$); 3.92 (2H, t, J=6Hz, $CH_2O$); 4.58 (1H, m, NHC*H*CO); 5.1 (2H, s, *$CH_2$*$C_6H_5$); 5.16 (1H, m, NH); 6.52 (1H, m, NH); 6.78 and 7.06 (each 2H, each d, each J=8Hz, $C_6H_4$); 7.3 (5H, m, $C_6H_5$); 7.74 (1H, m, NH).

The intermediate used in the preceeding section was prepared as described below:

(c) Tertiarybutyl-oxycarbonyl-O-3(methyl)butyl-L-tyrosine-N-methylamide

Tertiarybutyloxycarbonyl-L-tyrosine (14.1 g, 50 mmol) in dry dimethyl formamide (200 ml) was treated with sodium hydride (3.45 g, 115 mmol) under an atmosphere of argon at 10°C for 1 h. 3-Methylbutyl bromide (8.3 g, 55 mmol) was added and the reaction allowed to warm to 20°C over 20 h. Water (200 ml) was added and the solution washed with ethyl acetate (2 × 300 ml) and adjusted to pH 1 with 6N HCl. This solution was extracted with ethyl acetate and the extract dried over sodium sulphate and concentrated *in vacuo* to afford tertiary butyl oxycarbonyl-O-(3-methylbutyl)-L-tyrosine (15 g, 35 mmol) as a gum. The gum was dissolved in dichloromethane (200 ml) and treated with 1-hydroxybenzotriazole (5.8 g, 38 mmol) methylamine hydrochloride (2.2 g, 70 mmol), dicyclohexyl carbodiimide (7.8 g, 38 mmol), adjusted to pH 7 with N-methyl morpholine and stirred continuously as the reaction was allowed to warm from 0°C to 20°C over 16 h. The reaction was filtered and washed with water, aqueous saturated sodium hydrogen carbonate and aqueous saturated citric acid, dried over sodium sulphate and concentrated *in vacuo* to afford tertiarybutyloxycarbonyl-O-(3-methylbutyl)-L-tyrosine-N-methyl amide (6.45 g, 17 mmol) which crystallised from ethyl acetate/hexane as needles m.p. 115—116°C (Found: C, 66.3; H, 9.3; N, 8.0 $C_{20}H_{32}N_2O_4$ requires: C, 65.9; H, 8.9; N, 7.7%); δ($CDCl_3$) 0.94 (6H, d, J=6Hz, $(CH_3)_2C$); 1.42 (9H, s, $(CH_3)_3$); 1.6—2.0 (3H, m, $CH_2CH$) 2.74 (3H, d, J=4Hz, *$CH_3$*NH); 3.0 (2H, m, *$CH_2$*$C_6H_4$); 3.9 (2H, t, J=8Hz, $CH_2O$); 4.24 (1H, m, NHC*H*CO); 5.08 (1H, m, NH) 5.8 (1H, m, NH) 6.8, and 7.06 (each 2H, each d, each J=8Hz, $C_6H_4$).

Example 8

(a) N-[3-N-(Benzyloxycarbonyl)amino-1-(R)-carboxypropyl]-L-leucyl-O-(6-methylheptyl)-L-tyrosine N-methyl amide

N-[3-N-(Benzyloxycarbonyl)amino-1-(R)-methoxycarbonylpropyl]-L-leucyl-O-(6-methylheptyl)-L-tyrosine N-methyl amide (0.16 g, 0.24 mmol) in methanol (10 ml) was treated with aqueous sodium hyroxide (0.7 ml, 0.5M) with continuous stirring at 20°C for 24 h. The reaction was adjusted to pH 7 with acetic acid and concentrated *in vacuo* to afford a white solid which was washed with ethyl acetate and water and dried *in vacuo* to yield N-[3-N-(Benzyloxycarbonyl)amino-1-(R)-carboxypropyl]-L-leucyl-O-(6-methylheptyl)-L-tyrosine N-methylamide m.p. 176—177°C (Found: C, 66.3; H, 8.2; N, 8.4; $C_{36}H_{54}N_4O_7 + H_2O$ requires: C, 64.3; H, 8,4; N, 8.4%); δ($CDCl_3$) 0.84 (12H, m, 2 × $(CH_3)_2C$); 1.0—1.8 (14H, m, 6 × $CH_2$, 2 × CH); 2.56 (3H, d, J=$CH_3$NH); 2.4—3.64 (6H, m, 2 × $CH_2$, 2 × CH); 3.88 (2H, m, $CH_2O$); 4.82 (1H, m, NHC*H*CO); 5.0 (2H, s, $CH_2C_6H_5$); 6.74, and 7.06 (each 2H, each d, each J=8Hz, $C_6H_4$); 7.18 (1H, m, NH); 7.32 (5H, m, $C_6H_5$); 7.8, (1H, m, NH); 6.08 (1H, m, NH).

The methyl ester used in the preceeding section was prepared as described below:

(b) N-[3-N-(Benzoyloxycarbonyl)amino-1-(R)-methoxycarbonylpropyl]-L-leucyl-O-(6-methylheptyl)-L-tyrosine N-Methyl amide

Tertiarybutyloxycarbonyl-O-(6-methylheptyl)-L-tyrosine-N-methylamide (447 mg, 1.1 mmol) in $CH_2Cl_2$ (10 ml was treated with trifluoroacetic acid (10 ml) at 20°C for 2 h. The solvent was removed *in vacuo* and the residue redissolved in ether saturated with hydrogen chloride. This was repeated twice to afford O-(6-methylheptyl)-L-tyrosine-N-methylamide hydrochloride and treated with N-methyl morpholine (420 mg, 4.4 mmol), 1-hydroxybenzotriazole (159 mg, 1.04 mmol), N-[3-N-(Benzoyloxycarbonyl)amino-1-(R)-methoxycarbonylpropyl]-L-leucine hydrochloride (418 mg, 1 mmol) prepared as described in Example (1) and N-Ethyl-N'-3'dimethyl aminopropyl carbodiimide hydrochloride (200 mg, 1.04 mmol) at 0°C. The reaction was adjusted to pH 7 with N-methylmorpholine, stirred continuously and allowed to warm to 20°C over 1 h. The reaction was diluted with dichloromethane and washed successively with water, aqueous saturated sodium hydrogen carbonate and aqueous citric acid (1M) dried over sodium sulphate and concentrated *in vacuo* to afford a gum. Column chromatography on silica in ethyl acetate/hexane 1:1 afforded N-[3-N-(Benzyloxycarbonyl)amino-1-(R)-methoxycarbonylpropyl]-L-leucyl-O-(6-methylheptyl)-L-tyrosine N-methylamide (0.17 g, 0.25 mmol) as a colourless oil (Found: $[M+H]^+$ = 694.213. $C_{37}H_{56}N_4O_7$ requires: $[M+H]^+$ = 699.4227); δ($CDCl_3$) 0.88 (12H, m, 2 × $(CH_3)_2$CH); 1.12—1.92 (14H, m, 6 × $CH_2$, 2 ×CH) 2.74 (3H, d, J=4HzC*H*$_3$NH); 2.9—3.42 (6H, m, $CH_2$, 2 × CH); 3.64 (3H, s, $CH_3O$); 3.9 (2H, t, J=5HzC*H*$_2$NH); 4.6 (1H, q, NHC*H*CO); 5.1 (2H, s, $CH_2C_6H_5$); 5.3 (1H, m, NH); 6.7 (1H, m, NH); 6.76, and 7.06 (each 2H, each d, each J=8Hz, $C_6H_4$); 7.3 (5H, s, $C_6H_5$); 7.5 (1H, m, NH).

The preceeding intermediate was prepared as described below:

(c) N-Tertiary butyl-oxycarbonyl-O-(6-methylheptyl)-L-tyrosine-N-methylamide

N-Tertiarybutyloxycarbonyl-L-tyrosine (14.1 g, 50 mmol) in dry dimethylformamide (200 ml) was treated with sodium hydride (3.45 g, 115 mmol) at 10°C under an atmosphere of argon for 2 h. 6-Methylheptyl bromide (13.1 g, 50 mmol) was added and the reaction stirred at 20°C for 16 h. Water (200 ml) was added and the reaction extracted twice with ethyl acetate (400 ml). The aqueous phase was adjusted to pH 1 with hydrochloric acid (6M) and extracted twice with ethyl acetate (400 ml) and the ethyl acetate extracts combined, dried over sodium sulphate and concentrated *in vacuo* to a gum. Column chromatography on silica in ethyl acetate afforded crude N-tertiarybutyloxycarbonyl-L-tyrosine (4.9 g) as a colourless foam which was used directly in the next step. This acid (4.9 g) in dichloromethane (150 ml) was treated with methylamine hydrochloride (0.8 g, 25 mmol), 1-hydroxybenzotriazole (2.1 g, 14 mmol), dicyclohexylcarbodiimide (2.8 g, 14 mmol) adjusted to pH 7 with N-methylmorpholine at 0°C and stirred continuously as the reaction was allowed to warm to 20°C over 16 h. The reaction was filtered, washed with water, aqueous saturated sodium hydrogen carbonate and aqueous citric acid (1M), dried over sodium sulphate and concentrated *in vacuo* to a gum. Column chromatography on silica in ethyl acetate/hexane 1:1 afforded N-tertiarybutyloxycarbonyl-O-(6-methylheptyl)-L-tyrosine-N-methyl amide which crystallised from ethyl acetate as needles m.p. 103—106°C (Found: C, 67.5; H, 9.5; N, .9. $C_{23}H_{38}N_2O_4$ requires: C, 68.0; H, 9.4; N, 6.9%); δ($CDCl_3$) 0.88 (6H, d, J=6Hz, $(CH_3)_2C$); 1.12—1.86 (9H, m,$(CH_2)_4$CH); 1.4 (9H, s, $(CH_3)_3C$); 2.72 (3H, d, J=4Hz, C*H*$_3$NH); 2.98 (2H, m, C*H*$_2$$C_6H_4$); 3.9 (2H, t, J=6Hz, O—$CH_2$); 4.2 (1H, m, NHC*H*CO); 5.04 (1H, m, NH); 5.72 (1H, m, NH); 6.78, and 7.26 (each 2H, each d, each J=9Hz, $C_6H_4$).

## Example 9

(a) N-[3-N-(Benzyloxycarbonyl)amino-1-(R)-carboxypropyl]-L-leucyl-O-benzyl-L-tyrosine N-methylamide

To a stirred solution of the ester from example (0.36 g) in methanol (10 ml) was added dilute sodium hydroxide (1 ml 1M). After stirring for 48 h at room temperature the reaction mixture was neutralised with acetic acid and the product was filtered off. Recrystallisation from methanol/water gave the title compound as a solid (159 mg); mp. 172—175°; (Found: C, 65.70; H, 6.91; N, 8.40. $C_{35}H_{44}N_4O_7 \cdot 0.5\ H_2O$ requires C, 65.50; H, 7.06; N, 8.73%); δ($d^6$DMSO) 0.80 (6H, m, CH(C*H*$_3$)$_2$); 1.0—1.35 and 1.44—1.84 (together 5H, NHCH$_2$C*H*$_2$, C*H*$_2$C*H*(CH$_3$)$_2$); 2.4—3.5 (9H, NHC*H*$_2$CH$_2$C*H*CO$_2$H, α-CH, CH$_2$Tyr, CONHC*H*$_3$); 4.43 (1H, m, α-CH); 5.02 (2H, s, C*H*$_2$C$_6$H$_5$); 6.87 (2H, d, J=8Hz, Tyr); 7.11 (2H, d, J=8Hz, Tyr); 7.2—7.6 (11H, m, $C_6H_5$ × 2, CONH); 7.86 (1H, m, CONH); 8.16 (1H, d, J=8Hz, CONH).

(b) N-[3-N-(Benzyloxycarbonyl)amino-1-(R)-methoxycarbonylpropyl]-L-leucyl-O-benzyl-L-tyrosine N-Methylamide

To a cold (0°) solution of N-[3-N-(Benzyloxycarbonyl)amino-1-(R)-methoxycarbonylpropyl]-L-leucine (418 mg, 1 mmol) in dichloromethane (50 ml) was added N-methylmorpholine (0.1 g), 1-hydroxybenzotriazole (153 mg 1 mmol) and dicyclohexylcarbodiimide (206mg, 1 mmol). After stirring for 15 minutes at 0° O-benzyl L-tyrosine N-Methylamide hydrochloride (321 mg, 1 mmol) and N-methylmorpholine (0.1 g) were added. The reaction mixture after stirring and warming to room temperature over 4.5 h was filtered and washed with water, 3N citric acid, saturated aqueous sodium bicarbonate solution and water. The organic extract was dried and concentrated *in vacuo* to an oil. Column chromatography on silica eluting with 1:1 ethyl acetate/hexane in an increasing ethyl acetate gradient then gave the *title compound* as a foam (0.37 g); (Found: $[m+1]^+$ = 647.3417. $C_{36}H_{46}N_4O_7$ requires 647.3445); $\delta(CDCl_3)$ 0.88 (6H, m, CH($CH_3$)$_2$); 1.05—2.2 (6H, m, $NHCH_2$*$CH_2$*, *$CH_2CH$*$(CH_3)_2$); 2.74 (3H, d, J=5Hz, CONH*$CH_3$*); 2.96—3.44 (6H, m, NH*$CH_2$*$CH_2$*CH*$CO_2$, α-CH, $CH_2$Tyr); 3.66 (3H, s, $OCH_3$); 4.60 (1H, dd, J=7 and 15Hz, α-CH); 5.02 (2H, s, *$CH_2$*$C_6H_5$); 5.09 (2H, s, *$CH_2$*$C_6H_5$); 6.56 (1H, br, CONH); 6.90 (2H, d, J=8Hz, Tyr); 7.12 (2H, d, J=8Hz, Tyr); 7.25—7.6 (12H, m, CONH × 2, $C_6H_5$ × 2).

O-benzyl-L-tyrosine N-Methylamide hydrochloride used in this preparation was synthesised as follows:

N-tertiarybutoxycarbonyl-O-benzyl-L-tyrosine N-methylamide (3 g) was added to a mixture of TFA and $CH_2Cl_2$ (1:1 100 ml) at room temperature. After 15 minutes volatiles were removed *in vacuo* and the residue was dissolved in water. Neutralisation with solid sodium bicarbonate, extraction into $CH_2Cl_2$ and evaporation of the organic extract *in vacuo* then gave a solid. Co-evaporation of this with ethereal HCl gave O-benzyl-L-tyrosine N-Methylamide hydrochloride.

(c) N-Tertiarybutoxycarbonyl-O-benzyl-L-tyrosine N-methylamide

To a solution of N-tertiarybutoxycarbonyl-O-benzyl-L-tyrosine (7.4 g, 20 mmol), 1-hydroxybenzotriazole (3 g, 20 mmol), methylamine HCl (1.3 g, 20 mmol) and N-methylmorpholine (2 g, 20 mmol) in $CH_2Cl_2$ (20 ml) stirred and cooled to 0° was added DCC (4.2 g, 20 mmol). After being allowed to stir and warm to room temperature overnight the reaction mixture was filtered and washed with saturated aqueous sodium bicarbonate solution, 3N citric acid and brine. The dried organic extract was then concentrated *in vacuo* to give *N-tertiarybutoxycarbonyl-O-benzyl-L-tyrosine N-methylamide* as a solid. Recrystallisation from dichloromethane/hexane gave (4.5 g); m.p. 165—172°; (Found: C, 68.85; H, 7.43; N, 7.39. $C_{22}H_{28}N_2O_4$ requires C, 68.73; H, 7.34; N, 7.29%); $\delta(CDCl_3)$ 1.40 (9H, s, $OC(CH_3)_3$); 2.73 (3H, d, J=5Hz, CONH*$CH_3$*); 2.96 (2H, m, $CH_2$Tyr); 4.25 (1H, m, α-CH); 5.03 (2H, s, *$CH_2$*$C_6H_5$); 5.80 (1H, br, CONH); 6.86 (2H, d, J=8Hz, Tyr); 7.12 (2H, d, J=8Hz, Tyr); 7.3—7.5 (5H, m, $C_6H_5$).

## Example 10

(a) N-[3-N-(Benzyloxycarbonyl)amino-1-(R)-carboxypropyl]-L-leucyl-O-cyclopentyl-L-tyrosine N-Methylamide

To a solution of N-[3-N-(Benzyloxycarbonyl)amino-1-(R)-methoxycarbonylpropyl]-L-Leucyl-O-cyclopentyl-L-tyrosine N-Methylamide (536 mg, 0.86 mmol) in methanol (10 ml) was added dilute sodium hydroxide (1.8 ml, 1M). After stirring for 24 h at room temperature the reaction mixture was filtered, neutralised with acetic acid and concentrated *in vacuo* to a solid. Purification from ethyl acetate/water gave the *title compound* as a solid (245 mg); m.p. 160—170°C; (Found: C, 64.36; H, 7.71; N, 9.18. $C_{33}H_{46}N_4O_7$ + $0.25H_2O$ requires C, 64.42; H, 7.62; N, 9.11%); $\delta$($d^6$DMSO) 0.8 (6H, M, CH(*$CH_3$*)$_2$); 1.17 (2H, m, *$CH_2$*CH($CH_3$)); 1.45—2.0 (11H, m, $NHCH_2$*$CH_2$*, $CH_2$*CH*$(CH_3)_2$, CH-alkyl); 2.58 (3H, d, J=5Hz, CONH*$CH_3$*); 2.6—3.65 (6H, m, NH*$CH_2$*$CH_2$*CH*$CO_2$H, α-CH, $CH_2C_6H_4$); 4.42 (1H, q, J=7 and 15Hz, α-CH); 4.73 (1H, m, O—CH(cyclopentyl)); 5.02 (2H, s, *$CH_2$*$C_6H_5$); 6.77 (2H, d, J=8Hz, $C_6H_4$); 7.08 (2H, d, J=8Hz, $C_6H_4$); 7.25 (1H, br, CONH); 7.35 (5H, s, $C_6H_5$); 7.85 (1H, br, CONH); 8.15 (1H, d, J=Hz, CONH).

The preceeding ester was synthesised as described below:

(b) N-[3-N-(Benzyloxycarbonyl)amino-1-(R)-methoxycarbonylpropyl]-L-leucyl-O-cyclopentyl-L-tyrosine N-Methylamide

To a solution of N-[3-N-(Benzyloxycarbonyl)amino-1-(R)-methoxycarbonylpropyl]-L-leucine (418 mg, 1 mmol) in dichloromethane (10 ml) was added N-methylmorpholine (106 mg, 1 mmol) and the solution was stirred and cooled to 0°. 1-hydroxybenzotriazole (161 mg, 1.05 mmol), N-ethyl-N′-(3-dimethylaminopropyl)carbodiimide hydrochloride (202 mg, 1.05 mmol) and N-methylmorpholine (106 mg, 1 mmol) were then added. After 15 minutes a solution of O-cyclopentyl-L-tyrosine N-methylamide (1.1 mmol) in dichloromethane (5 ml) (which had been pepared by treatment of the N-tertiarybutoxycarbonyl precursor with trifluroacetic acid and subsequent neutralisation with N-methylmorpholine) was added. The reaction after being allowed to stir and warm to room temperature overnight was diluted with dichloromethane (30 ml), washed with water, saturated aqueous sodium bicarbonate and water. The dried organic extract was concentrated *in vacuo* to give the *title compound* as an oil (0.55 g); (Found: $[m+1]^+$ = 625.3608. $C_{34}H_{49}N_4O_7$ requires 625.3601); $\delta(CDCl_3)$ 0.82 (6H, m, CH(*$CH_3$*)$_2$); 1.0—2.0 (11H, m, *$CH_2CH$*$(CH_3)_2$, CH-cyclopentyl); 2.7(3H, d, J=Hz, CONH*$CH_3$*); 2.9—3.5 (6H, m, NH*$CH_2$*$CH_2$*CH*$CO_2$H, α-CH, CH2Tyr); 3.65 (3H, s, $OCH_3$); 4.58

(1H, dd, J=7 and 15Hz, α-CH); 4.68 (1H, br, ?); 5.08 (2H, s, *$CH_2$*$C_6H_5$); 5.22 (1H, br, CONH); 6.68 (1H, br, CONH); 6.78 (2H, d, J=8Hz, Tyr); 7.10 (2H, d, J=8Hz, Tyr); 7.32 (5H, s, $C_6H_5$); 7.52 (1H, d, J=8Hz, CONH).

The N-(Tertiarybutyoxycarbonyl)-O-cyclopentyl-L-tyrosine N-Methylamide used in this preparation was prepared as follows:

(c) N-(Tertiarybutoxycarbonyl)-O-cyclopentyl-L-tyrosine N-Methylamide

To a cold (0°) solution of N-(Tertiarybutoxycarbonyl)-O-cyclopentyl-L-tyrosine (6.89 g, 20 mmol) in dichloromethane (40 ml) was added 1-hydroxybenzotriazole (3.32 g, 21 mmol) and dicyclohexylcarbodiimide (4.47 g, 21 mmol). After 20 minutes at 0° a solution of methylamine (1.34 g, 40 mmol) in dichloromethane (20 ml) was added and the reaction mixture was allowed to stir and warm to room temperature overnight. The reaction mixture after filtration and washing with water, dilute (1M) sodium hydroxide, 3N citric acid and water was dried and concentrated *in vacuo* to an oil. Recrystallisation from ethyl acetate/hexane gave the *title compound* as white crystals (1 g); m.p. 152—154°; (Found: C, 66.77; H, 8.47; N, 7.83. $C_{20}H_{30}N_2O_4$ requires C, 66.27; H, 8.34; N, 7.73%); δ($CDCl_3$) 1.40 (9H, s, $C(CH_3)_3$); 1.5—2.0 (8H, CH-cyclopentyl); 2.72 (3H, d, J=5Hz, CONH*$CH_3$*); 2.95 (2H, m, $CH_2$Tyr); 4.22 (1H, dd, J=7 and 15Hz, α-CH); 4.72 (1H, br, O—CH (cyclopentyl)); 5.07 (1H, br, CONH); 5.75 (1H, br, CONH); 6.78 (2H, d, J=8Hz, $C_6H_4$); 7.10 (2H, d, J=8Hz, $C_6H_4$).

The N-(Tertiarybutoxycarbonyl)-O-cyclopentyl-L-tyrosine used in this preparation was prepared as follows:

(d) N-(Tertiarybutoxycarbonyl)-O-cyclopentyl-L-tyrosine

To a cold (10°) stirred solution of N-tertiarybutoxycarbonyl-L-tyrosine (11.25 g, 40 mmol) in dry DMF (150 ml) under argon was added sodium hydride (2.76 g, 80% dispersion in oil, 92 mmol). After one hour at 10° cyclopentyl bromide (5.96 g, 40 mmol) was added and the reaction was allowed to warm and stir to room temperature overnight. Water (200 ml) and dichloromethane (300 ml) were then added and the aqueous layer was separated. The separated aqueous layer was washed with dichloromethane, acidified with hydrochloric acid and re-extracted with dichloromethane (300 ml × 2). These latter dichloromethane extracts were dried and concentrated *in vacuo* to an oil. Column chromatography of this material on silica eluting with ethyl acetate gave the *title compound* as a foam (7.05 g). This was used directly in the next step without further purification.

Example 11

(a) N-[3-N-(Benzyloxycarbonyl)amino-1-(R)-carboxypropyl]-L-leucyl-O-(2-butylamino-2-oxo-ethyl)-L-tyrosine N-Methylamide

To a solution of N-[3-N-(Benzyloxycarbonyl)amino-1-(R)-methoxycarbonylpropyl]-L-leucyl-O-(2-butylamino-2-oxo-ethyl)-L-tyrosine N-Methylamide (199 mg, 0.3 mmol) in methanol (10 ml) was added dilute sodium hydroxide (0.5 ml, 1M). After stirring for 90 h at room temperature the reaction mixture was neutralised with acetic acid and concentrated *in vacuo*. Purification from ethyl acetate/water gave the *title compound* as a solid (143 mg); m.p. 164—170°C; (Found: C, 59.44; H, 7.39; N, 10.49. $C_{34}H_{49}N_5O_8$ + 1.5 $H_2O$ requires C, 59.81; H, 7.68; N, 10.26%); δ($d^6$DMSO) 0.7—1.0 (9H, m, $CH_2$*$CH_3$*, CH(*$CH_3$*)$_2$); 1.2—2.0 (9H, m, $NHCH_2$*$CH_2$*, $NHCH_2$(*$CH_2$*)$_2CH_3$, *$CH_2CH$*($CH_3$)); 2.62—3.64 (9H, m, NH*$CH_2$*$CH_2$*CH*$CO_2H$, CH, $CH_2$Tyr, CONH*$CH_3$*; 4.64 (2H, s, $OCH_2CO$); 4.56 (1H, m, α-CH); 5.07 (2H, s, *$CH_2$*$C_6H_5$); 6.90 (2H, d, J=8Hz, Tyr); 7.17 (2H, d, J=8Hz, Tyr); 7.34 (5H, br, s, $C_6H_5$).

(b) N-[3-N-(Benzyloxycarbonyl)amino-1-(R)-methoxycarbonylpropyl]-L-leucyl-O-(2-butylamino-2-oxo-ethyl)-L-tyrosine N-Methylamide

To a cold (0°) stirred solution of N-[3-N-(Benzyloxycarbonyl)amino-1-(R)-methoxycarbonylpropyl]-L-Leucyl-O-Carboxymethyltyrosine N-Methylamide HCl in $CH_2Cl_2$/DMF (11 ml, 10:1) was added N-methylmorpholine (240 mg, 2.4 mmol), 1-hydroxybenzotriazole (180 mg, 1.2 mmol) and N-ethyl-N'-(3-dimethylaminopropyl)arbodiimide hydrochloride (0.24 g, 1.2 mmol). After stirring for 15 min. at 0° a solution of n-butylamine (90 mg, 1.2 mmol) in $CH_2Cl_2$ was added and the reaction mixture was allowed to warm and stir to room temperature overnight. The reaction mixture after dilution with $CH_2Cl_2$ (30 ml) was washed (water (10 ml), 3N citric acid (10 ml) and saturated aqueous sodium bicarbonate (10 ml)), dried, filtered and concentrated *in vacuo* to an oil. Crystallisation from ethyl acetate/hexane then gave the *title compound* as a white solid (0.27 g); m.p. 101—107°; (Found: C, 62.13; H, 7.84; N, 10.42. $C_{35}H_{51}N_5O_8$ + 0.5 $H_2O$ requires C, 61.93; H, 7.72; N, 10.32%); δ($CDCl_3$) 0.87 (6H, m, $CH_2CH$(*$CH_3$*)$_2$); 0.94 (3H, t, J=7Hz, $CH_2$*$CH_3$*); 1.04—2.0 (9H, m, $NHCH_2$*$CH_2$*, *$CH_2CH$*$(CH_3)_2$, $NHCH_2$(*$CH_2$*)$_2CH_3$); 2.75 (3H, d, J=5Hz, CONH*$CH_3$*); 2.85—3.45 (9H, M, NH*$CH_2$*$CH_2$*CH*$CO_2H$, NH*$CH_2$*, α-CH, $CH_2$Tyr); 3.66 (3H, s, $OCH_3$); 4.42 (2H, s, $OCH_2CO$); 4.63 (1H, dd, J=7 and 15Hz, α-CH); 5.07 (2H, s, *$CH_2$*$C_6H_5$); 5.26 (1H, br, CONH); 6.63 (1H, br, CONH); 6.82 (2H, d, J=8Hz, Tyr); 7.15 (2H, d, J=8Hz, Tyr); 7.34 (5H, s, $C_6H_5$); 7.58 (1H, d, J=10Hz, CONH).

Example 12

(a) N-[3-N-(Benzyloxycarbonyl)amino-1-(R)-carboxypropyl]-L-leucyl-O-(2'-carboxyethyl-L-leucyl N-Methylamide)-L-tyrosine N-Methylamide

To a solution of N-[3-N-(Benzyloxycarbonyl)amino-1-(R)-methoxycarbonylpropyl]-L-leucyl-O-(2'-carboxyethyl-L-leucyl N-Methylamide)-L-tyrosine N-Methylamide (300 mg, 0.4 mmol) in methanol (10 ml) was added dilute sodium hydroxide (1.2 ml, 0.5M). After stirring for 48 h at room temperature the reaction mixture was neutralised with acetic acid and concentrated *in vacuo*. Recrystallisation from methanol/water gave the *title compound* as a solid (115 mg); m.p. 162—163°; (Found: C, 60,26; H, 7.49; N, 11.09 $C_{37}H_{54}N_6O_9$ requires C, 60.39; H, 7.53; N, 11.42%); δ($d^6$DMSO) 0.7—0.9 (12H, m, CH($CH_3$)$_2$ × 2); 0.95—1.85 (8H, m, $CH_2CH$($CH_3$) × 2, NHCH$_2$$CH_2$); 2.45—3.7 (13H, m, NH$CH_2$CH$_2$$CH$CO$_2$H, CONH$CH_3$ × 2, α-CH × 2, CH$_2$Tyr); 4.34 (1H, m, —CH); 4.46 (2H, s, OCH$_2$CO); 5.05 (2H, s, $CH_2$C$_6$H$_5$); 6.82 (2H, d, J=8Hz, Tyr); 7.13 (2H, d, J=8Hz, Tyr); 7.24 (1H, br, CONH); 7.35 (5H, s, $C_6H_5$); 7.85 (1H, br, CONH); 7.98 (2H, br, CONH × 2); 8.15 (1H, d, J=10Hz, CONH).

The ester required in this preparation was prepared as follows:

(b) N-[3-N-(Benzyloxycarbonyl)amino-1-(R)-methoxycarbonylpropyl]-L-leucyl-O-(2′-carboxyethyl-L-leucyl N-Methylamide)-L-tyrosine N-Methylamide

To a cold (0°) solution of N-[3-N-(Benzyloxycarbonyl)amino-1-(R)-methoxycarbonylpropyl]-L-leucyl-O-(2′-carboxyethyl)-L-tyrosine N-Methylamide (0.6 g, 1.1 mmol) in $CH_2Cl_2$/DMF (11 ml 10:1) was added 1-hydroxybenzotriazole (0.18 g, 1.2 mmol), N-ethyl-N′-(3-dimethylaminopropyl)carbodiimide hydrochloride (0.24 g, 1.2 mmol) and N-methylmorpholine (0.24 g, 2.4 mmol). After 5 min. at 0° L-leucine N-methylamide HCl (0.22 g, 1.2 mmol) and N-methylmorpholine (120 mg, 1.2 mmol) were added and the reaction was allowed to stir and warm to room temperature overnight. The reaction mixture after diluting with $CH_2Cl_2$ (30 ml) was washed with water (20 ml), 3N citric acid (30 ml) and saturated aqueous sodium bicarbonate solution. The organic extract was dried and concentrated *in vacuo* to an oil. Recrystallisation from ethyl acetate/hexane gave the *title compound* as crystals (350 mg); m.p. 122—123°; (Found: C, 61.16; H, 7.45; N, 11.14. $C_{38}H_{56}N_6O_9$ requires C, 61.60; H, 7.62; N, 11.34%); δ($CDCl_3$) 0.8—1.0 (12H, m, CH($CH_3$)$_2$ × 2); 1.05—2.05 (8H, m, $CH_2CH$(CH$_3$)$_2$ × 2, NHCH$_2$$CH_2$); 2.75 (3H, d, J=5Hz, CONH$CH_3$); 2.80 (3H, d, J=5Hz, CONH$CH_3$); 2.8—3.5 (7H, m, NH$CH_2$CH$_2$$CH$CO$_2$, CH$_2$Tyr, α-CH × 2); 3.68 (3H, s, OCH$_3$); 4.44 (2H, s, OCH$_2$CO); 4.4—4.7 (2H, m, α-CH × 2); 5.10 (2H, s, $CH_2$C$_6$H$_5$); 5.34 (1H, t, J=6Hz, CONH); 6.42 (1H, br, CONH); 6.73 (1H, br, CONH); 6.82 (2H, d, J=8Hz, Tyr); 7.0 (1H, br, CONH); 7.15 (2H, d, J=8Hz, Tyr), 7.36 (5H, s, $C_6H_5$); 7.56 (1H, br, CONH).

## Example 13

(a) N-[3-N-(Benzyloxycarbonyl)amino-1-(R)-carboxypropyl]-L-leucyl-O-methyl-L-tyrosine N-Phenethylamide

To a solution of N-[3-(Benzyloxycarbonyl)amino-1-(R)-methoxycarbonylpropyl]-L-leucyl-O-methyl-L-tyrosine N-Phenethylamide (210 mg, 0.33 mmol) in methanol (5 ml) was added dilute sodium hydroxide (0.5 ml, 1M). After stirring for 5 h at room temperature the reaction mixture was concentrated *in vacuo* and diluted with water. The aqueous extract after washing with ether was acidified to pH 3 with dilute (1N) HCl. The resulting solid was filtered and dried to give the *tile compound* as a solid (110 mg); (Found: C, 65.5; H, 7.0; N, 9.1. · 0.5 $H_2O$ requires C, 65.5; H, 7.0; N, 8.7%).

(b) N-[3-N-(Benzyloxycarbonyl)amino-1-(R)-methoxycarbonylpropyl]-L-leucyl-O-methyl-L-tyrosine N-Phenethylamide

To a cold (0°) solution of N-[3-N-(Benzyloxycarbonyl)amino-1-(R)-methoxycarbonylpropyl]-L-leucyl-O-methyl-L-tyrosine Hydrochloride (400 mg, 0.67 mmol) in DMF was added N-methylmorpholine (68 mg, 0.67 mmol), 1-hydroxybenzotriazole (107 mg, 0.7 mmol) and benzylamine (72 mg, 0.67 mmol). The reaction mixture was then cooled to −10° and N-ethyl-N′-(3-dimethylaminopropyl)carbodiimide hydrochloride (134 mg, 0.7 mmol) and N-methylmorpholine (71 mg, 0.7 mmol) were added. After being allowed to warm and stir to room temperature over 20 h the reaction mixture was concentrated *in vacuo*. The resultant residue after dissolving in $CH_2Cl_2$ and washing (water, saturated aqueous sodium bicarbonate solution) was concentrated *in vacuo*. Recrystallisation from ethyl acetate/hexane gave the *title compound* (290 mg); (Found: C, 66.9; H, 7.1; N, 8.7. required C, 67.2; H, 7.3; N, 8.6%); δ($CDCl_3$) 0.86 (6H, M, CH($CH_3$)$_2$); 1.1—1.9 (6H, m, N$H$CH$_2$$CH_2$, $CH_2CH$(CH$_3$)$_2$); 2.95—3.42 (6H, m, NH$CH_2$CH$_2$$CH$CO$_2$CH$_3$, α-CH, CH$_2$Tyr); 3.45 (3H, s, OCH$_3$); 3.73 (3H, s, OCH$_3$); 4.38 (2H, m, $CH_2$C$_6$H$_5$); 4.65 (1H, q, J=7 and 15Hz, α-CH); 5.04 (1H, br, CONH); 5.08 (2H, s, $CH_2$C$_6$H$_5$); 6.78 (2H, d, J=8Hz, Tyr); 6.96 (1H, br, CONH); 7.11 (2H, d, J=8Hz, Tyr); 7.10-7.30 (5H, m, $C_6H_5$); 7.34 (5H, s, $C_6H_5$); 7.54 (1H, d, J=8Hz, CONH).

The starting material for this reaction was prepared as follows:

(c) N-[3-N-(Benzyloxycarbonyl)amino-1-(R)-methoxycarbonylpropyl]-L-leucyl-O-methyl-L-tyrosine

N-[3-N-(Benzyloxycarbonyl)amino-1-(R)-methoxycarbonylpropyl]-L-leucyl-O-methyl-L-tyrosine t-butyl ester (3 g) was dissolved in TFA (30 ml) and water (1.5 ml). After 4 h at room temperature volatiles were removed *in vacuo* and the residue was co-evaporated twice with toluene. Trituation of the resulting solid with ethereal HCl then gave N-[3-N-(Benzyloxycarbonyl)amino-1-(R)-methoxycarbonylpropyl]-L-leucyl-O-methyl-L-tyrosine as its hydrochloride salt.

(d) N-[3-N-(Benzyloxycarbonyl)amino-1-(R)-methoxycarbonylpropyl]-L-leucyl-O-methyl-L-tyrosine ᵗButyl ester

A solution of methyl 4-N-(benzyloxycarbonyl)amino-2-bromo-butanoate (2.5 g, 7.5 mmol), L-leucyl-O-methyl-L-tyrosine t-butyl ester (1.82 g, 5 mmol), (which had been prepared by hydrogenolysis of the N-benzyloxycarbonyl precursor in the usual manner), and N-methylmorpholine (0.76 g, 7.5 mmol) in acetonitrile (15 ml) was heated under reflux for 30 h. The reaction mixture was then concentrated *in vacuo*, dissolved in $CH_2Cl_2$, washed (water, 3N citric acid and saturated aqueous sodium bicarbonate solution), dried and evaporated *in vacuo* to yield an oil. Column chromatography on silica eluting with 40:60 ethyl acetate/hexane gave the *title compound* as an oil (670 mg); δ($CDCl_3$) 0.88 (6H, dd, J=7Hz, $CH(CH_3)_2$); 1.42 (9H, s, $OC(CH_3)_3$); 1.62 (3H, m, $CH_2CH(CH_3)_2$; 1.86 (2H, m, $NHCH_2CH_2$); 2.9—3.36 (6H, m, $NHCH_2CH_2CHCO_2CH_3$, α-CH, $CH_2$Tyr); 3.66 (3H, s, $OCH_3$); 3.78 (3H, s, $OCH_3$); 4.68 (1H, dd, J=7 and 15Hz, α-CH); 5.09 (2H, s, $CH_2C_6H_5$); 5.16 (1H, br, CONH); 6.80 (2H, d, J=8Hz, Tyr); 7.01 (1H, br, CONH); 7.07 (2H, d, J=8Hz, Tyr); 7.35 (5H, s, $C_6H_5$).

The N-(benzyloxycarbonyl)-L-leucyl-O-methyl-L-tyrosine t-butyl ester used in this preparation was prepared as follows:

(e) N-(Benzyloxycarbonyl)-L-leucyl-O-methyl-L-tyrosine ᵗButyl ester

To a cold (0°) solution of N-tertiarybutoxycarbonyl-L-leucine (4 g, 15.1 mmol), O-methyl-L-tyrosine t-butyl ester (3.8 g, 15.1 mmol) and 1-hydroxybenzotriazole (2.43 g, 15.9 mmol) in DMF (30 ml) was added N-methylmorpholine (1.61 g, 15.9 mmol) and N-ethyl-N'-(3-dimethylaminopropyl)carbodiimide hydrochloride (3.04 g, 15.9 mmol). After stirring at 0° for 1 h the solution was allowed to warm to room temperature overnight. The reaction mixture was then concentrated *in vacuo*, dissolved in ethyl acetate, washed (water, 0.5 mole citric acid, brine, saturated aqueous sodium bicarbonate solution and brine), dried and evaporated *in vacuo* to give an oil. Chromatography on silica eluting with 25% ethyl acetate in hexane then gave the *title compound* as a foam (4.65 g); (Found: $[m+1]^+$ = 499.2803. $C_{28}H_{39}N_2O_6$ requires 499.2808); δ($CDCl_3$) 0.92 (6H, m, $CH(CH_3)_3$); 1.42 (9H, s, $OC(CH_3)_3$); 1.4—1.83 (3H, m, $CH_2CH(CH_3)_2$); 3.03 (2H, m, $CH_2$Tyr); 3.76 (3H, s, $OCH_3$); 4.18 (1H, m, α-CH); 4.69 (1H, dd, J=7 and 15Hz, α-CH); 5.12 (2H, s, $CH_2C_6H_5$); 5.17 (1H, m, CONH); 6.43 (1H, m, CONH); 6.79 (2H, d, J=8Hz, Tyr); 7.06 (2H, d, J=8Hz, Tyr); 7.36 (5H, s, $C_6H_5$).

The O-methyl-L-tyrosine t-butyl ester used in this synthesis was prepared as follows:

(f) O-methyl-L-tyrosine t-butyl ester

To a cold, (−5°), stirred solution of N-benzyloxycarbonyl-O-methyl-L-tyrosine (6.58 g, 20 mmol) in t-butanol/pyridine (70 ml, 5:2) was added phosphorus oxychloride (3.35 g, 22 mmol) dropwise over 0.5 h. After a further 0.5 h at −5° the reaction was warmed to room temperature and was then left to stir overnight. The reaction mixture, after diluting with ethyl acetate and washing (0.5M citric acid, brine, saturated aqueous sodium bicarbonate solution and brine) was concentrated *in vacuo* to give the required t-butyl ester (7.54 g). To a portion of this (7.4 g, 19.2 mmol) in methanol (100 ml) was added 10% Pd/C (1 g) and acetic acid (1.16 g, 19.2 mmol). After stirring the reaction mixture under hydrogen at room temperature for 4 h it was filtered and evaporated *in vacuo*. The residue was then taken up in dichloromethane and saturated aqueous sodium bicarbonate solution and the organic phase was separated, washed with brine, dried and evaporated *in vacuo* to yield O-methyl-L-tyrosine t-butyl ester as a solid (3.95 g).

The activities of representative compounds according to the invention are given below in Table I.

TABLE I

| Example No. | $IC_{50}$ (μM) Human Rheumatoid Synovial Collagenase |
|---|---|
| 1 | 0.81 |
| 2 | 1.2 |
| 3 | 0.74 |
| 4 | 2.1 |
| 5 | 3.6 |
| 6 | 0.54 |
| 7 | 1.1 |
| 8 | 2.5 |
| 9 | 0.91 |
| 10 | 0.74 |
| 11 | 2.6 |
| 12 | 2.4 |
| 13 | 1.0 |

One of the preferred compounds in accordance with the present invention was tested *in vivo* to demonstrate the antiarthritic activity thereof in a standardized animal model test and compared against prednisolone which has been shown to be clinically effective in the treatment of rheumatoid arthritis.

The therapeutic effectiveness is assessed in the rat model of inflammatory arthritis described by Trentham et al. (1977). In this model, the arthritis, induced by an intradermal injection of Type II collagen, generally develops in both hind paws and the progression and severity of the lesion are monitored by physical measurements of the malleolar width and ankle extensibility.

The compound of example II in the present specification was administered by intraperitoneal injections at 100 mg/kg twice daily for 21 days commencing seven days after the injection of the disease.

The results of the foregoing test are set forth in Table III below.

TABLE II

The effect of N-[3-N-(benzyloxycarbonyl)amino-1-(R)-carboxypropyl]-L-leucyl-O-methyl-L-tyrosine-N-methylamide (Example 1) on the Physical Parameters of Arthritic Rats' Paws.

| PARAMETER | PERCENTAGE CHANGE FROM CONTROL EXPERIMENT NUMBER 1 | 2 | 3 | MEAN x |
|---|---|---|---|---|
| MALLEOLAR WIDTH | | | | |
| Example 1 | 65 | 43 | 41 | 50 |
| Prednisolone | 98 | 96 | 97 | 97 |
| ANGLE EXTENSION | | | | |
| Example 1 | 66 | 39 | 39 | 48 |
| Prednisolone | 100 | 96 | 95 | 97 |

Drugs (Example 1 or prednisolone) and vehicle were administered to groups of 10 rats each. Physical measurements on the rat paws were made weekly for four weeks and consisted of (a) the distance between the medial and lateral malleolus (malleolar width) and (b) the ankle extension which was measured by applying a standard force to the rat paw and measuring the angle formed between the flexor surface of the foot and tibia. Evidence of a therapeutic effect was denoted by a significant reduction in malleolar width and a significant reduction in malleolar width and a significant increase in ankle extension. Both measurements were carried out on apparatus designed for the purpose. Area-under-the-curve data from these time course experiments was examined by non-parametric statistical analysis. The prednisolone standard was given at a dose of 0.625 mg/kg b.i.d. Prednisolone was chosen as a standard since steroids are known to be effective both clinically in Rheumatoid Arthritis and in animal models of arthritis.

References

Trentham, D.E., Townes, A. S. & Kang, A. H. (1977) "Autoimmunity to Type II Collagen: An Experimental Model of Arthritis" J. Expt. Med. *146.* 857—868.

**Claims**

1. A compound of the formula:

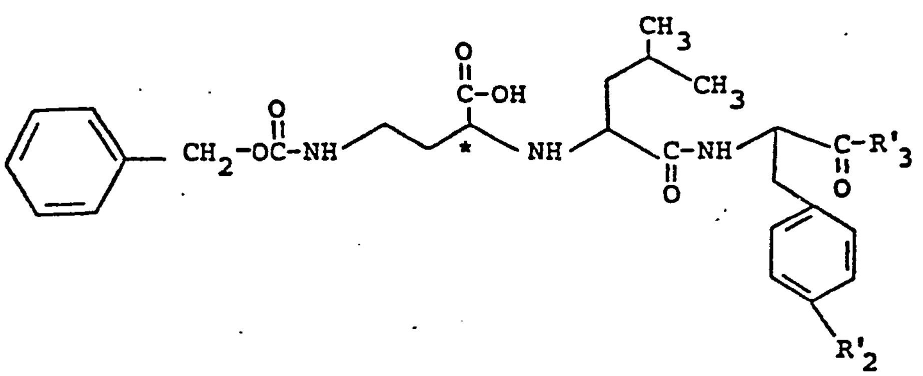

and the pharmaceutically acceptable acid addition salts thereof wherein R'$_2$ represents hydroxy, straight or branched chain alkoxy of from 1 to 10 carbon atoms, cycloalkoxy of from 3 to 10 carbon atoms, aralkoxy comprising phenyl or naphthyl and an alkyl portion of from 1 to 4 carbon atoms or substituted alkoxy wherein the substituent is selected from alkylaminocarbonyl or the group

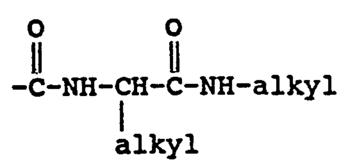

and R'$_3$ represents methylamino or phenylethylamino; and the stereochemistry of the carbon atom marked by the asterisk is R or S or a diastereometric mixture thereof, excluding N-(3-N-(benzyloxycarbonyl)amino-1-(R)-carboxypropyl)-L-leucyl-O-methyl-L-tyrosine N-methylamide.

2. A compound according to Claim 1 wherein $R'_2$ is selected from n-propoxy or n-pentoxy.

3. A compound according to Claim 1 wherein the stereochemistry of the carbon atom marked by the asterisk is R.

4. A compound according to Claim 1 of the formula:

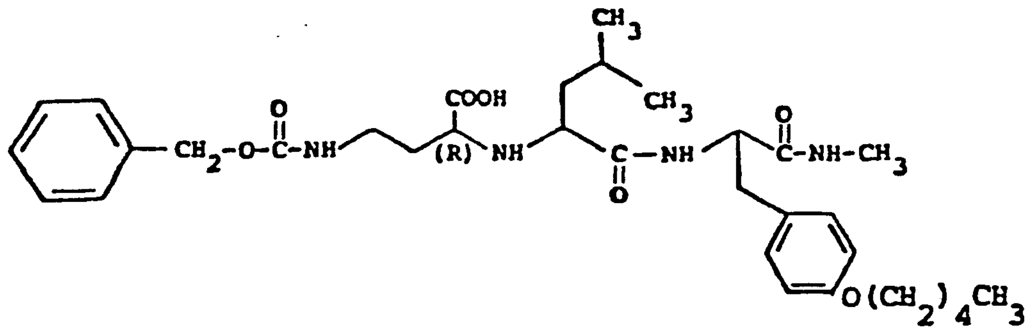

or a pharmaceutically acceptable acid addition salt thereof.

5. A compound according to Claim 1 of the formula:

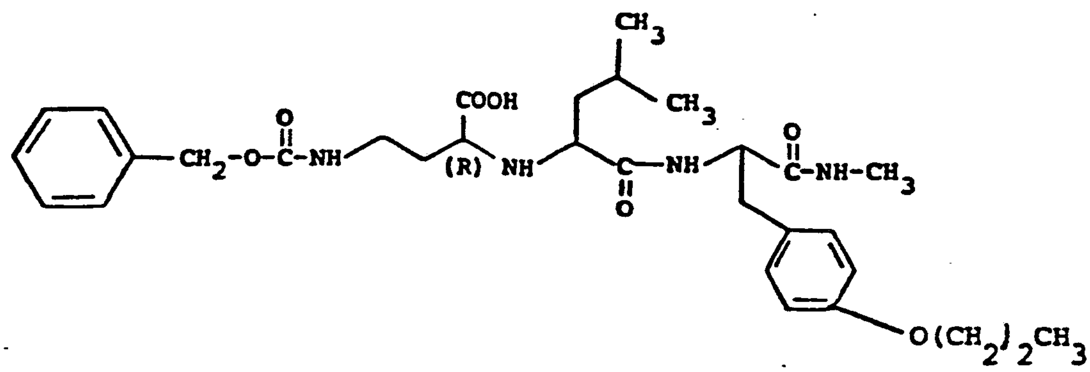

or a pharmaceutically acceptable acid addition salt thereof.

**Patentansprüche**

1. Verbindung der Formel

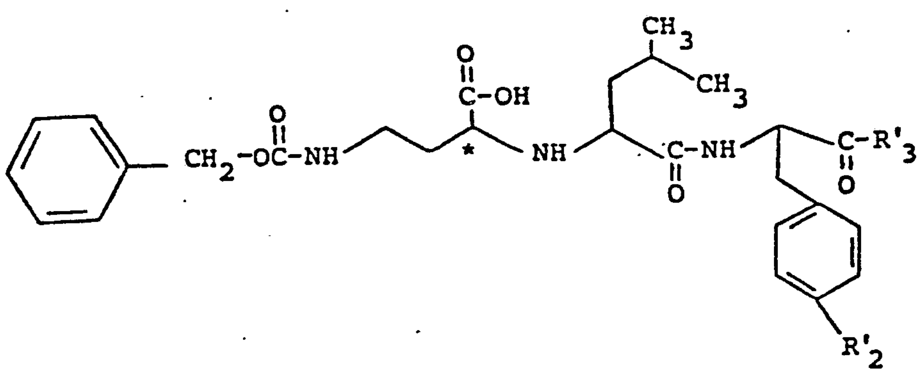

und deren pharmazeutisch annehmbare Säureadditionssalze, worin $R'_2$ Hydroxy, geradkettiges oder kettenverzweigtes Alkoxy mit 1 bis 10 Kohlenstoff-Atomen, Cycloalkoxy mit 3 bis 10 Kohlenstoff-Atomen, Aralkoxy mit Phenyl oder Naphthyl und einem Alkyl-Teil mit 1 bis 4 Kohlenstoff-Atomen oder substituiertes Alkoxy darstellt, worin der Substituent aus Alkylaminocarbonyl oder der Gruppe

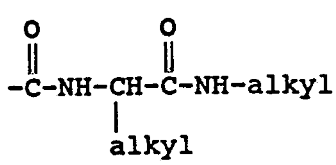

ausgewählt ist und $R'_3$ Methylamino oder Phenylethylamino darstellt; und die Stereochemie des durch das Sternchen bezeichneten Kohlenstoff-Atoms R oder S ist, oder ein diastereomeres Gemisch derselben, ausgenommen N-[3-N-(benzyloxycarbonyl)amino-1-(R)-carboxypropyl]-L-leucyl-O-methyl-L-tyrosin N-methylamid.

2. Verbindung nach Anspruch 1, worin $R'_2$ aus n-Propoxy oder n-Pentoxy ausgewählt ist.

3. Verbindung nach Anspruch 1, worin die Stereochemie des durch das Sternchen bezeichneten Kohlenstoff-Atoms R ist.

4. Verbindung nach Anspruch 1 der Formel

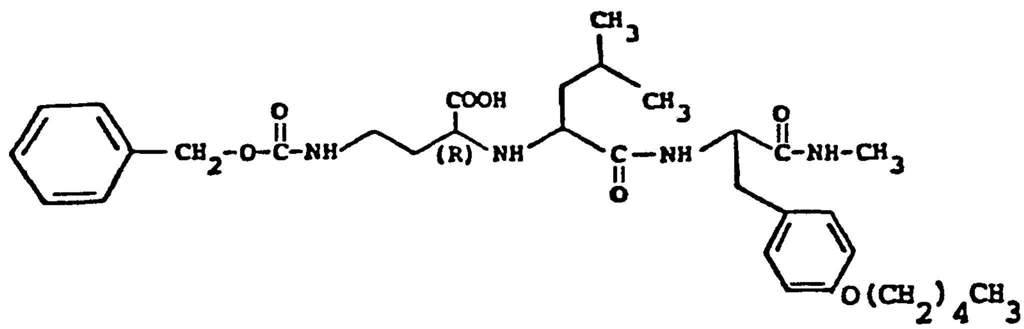

oder deren pharmazeutisch annehmbares Säureadditionssalze.

5. Verbindung nach Anspruch 1 der Formel

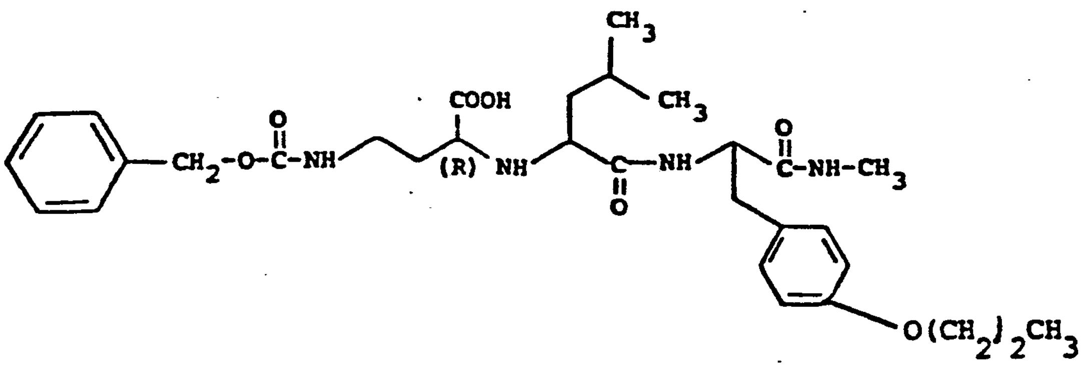

oder deren pharmazeutisch annehmbares Säureadditionssalz.

**Revendications**

1. Un composé de formule:

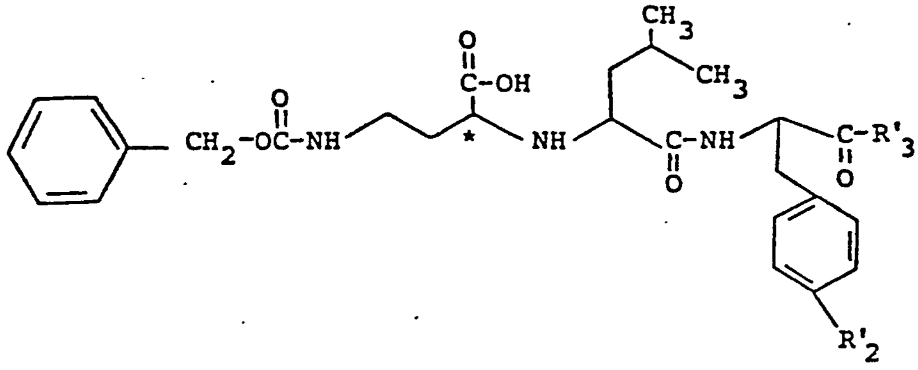

et ses sels d'addition d'acides pharmaceutiquement acceptables, dans laquelle $R'_2$ représente un groupe hydroxy, une group alcoxy en $C_1$—$C_{10}$ à chaîne droite ou ramifiée, un groupe cycloalcoxy en $C_3$—$C_{10}$, un

groupe arylalcoxy comprenant un reste phényle ou naphtyle et une portion alkyle en en $C_1$—$C_4$ ou un reste alcoxy substitué dans lequel le substituant est choisi parmi un groupe alkylaminocarbonyle ou le groupe

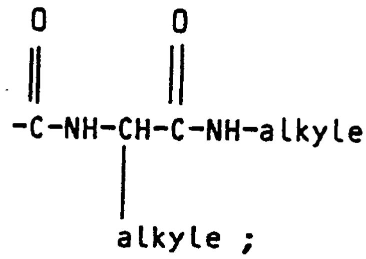

et R'$_3$ représente un groupe méthylamino ou phényléthylamino; et la stéréochimie de l'atome de carbone marqué par un astérisque est R ou S, ou un mélange de ces deux diastéréomères, à l'exclusion du N-méthylamide de N-(3-N-(benzoxycarbonyl)amino-1-(R)-carboxypropyl)-L-leucyl-O-méthyl-L-tyrosine.

2. Un composé selon la revendication 1, dans lequel R'$_2$ est un groupe n-propoxy ou n-pentoxy.

3. Un composé selon la revendication 1, dans lequel la stéréochimie de l'atome de carbone marqué par un astérisque est R.

4. Un composé selon la revendication 1, de formule:

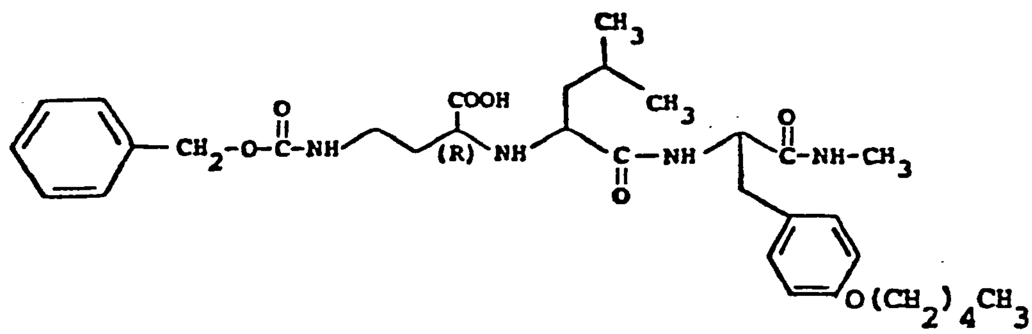

ou un de ses sels d'addition d'acides pharmaceutiquement acceptables.

5. Un composé selon la revendication 1, de formule:

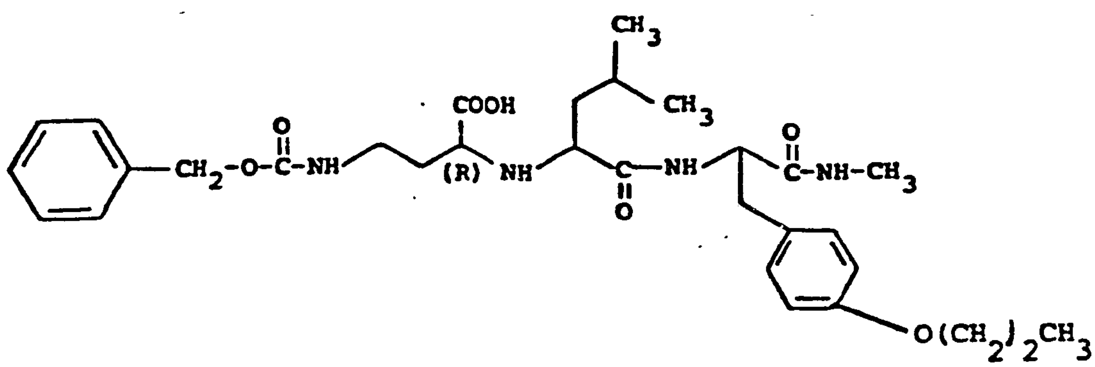

ou un de ses sels d'addtiion d'acides pharmaceutiquement acceptables.